# EUROPEAN PATENT APPLICATION

(11) **EP 4 119 927 A1**
(43) Date of publication of application: **18.01.2023**
(21) Application number: 22193915.0
(22) Date of filing: 02.07.2013
(51) Int. Cl.: G01N 21/07, B01L 3/00, G01J 1/26, G01J 1/42

(54) **TEST DEVICE AND CONTROL METHOD THEREOF**

(30) Priority: 11.07.2012 KR 20120075302
(62) Divisional of application: 13174639.8
(71) Applicant: Nexus Dx, Inc., San Diego, CA 92121 (US)
(72) Inventor: KIM, Jong Cheol, 101-1106 Seoul (KR)
(74) Representative: Kilger, Ute

(57) **Abstract**

A test device to detect a detection object (20) of a microfluidic device (10) is provided. The test device includes a light emitting element (1333) configured to emit light onto the microfluidic device, a light receiving element (1411) configured to capture an image of the detection object through light emitted from the light emitting element, and a controller (1200) configured to control operations in the test device.

## Description

Apparatuses and methods consistent with exemplary embodiments relate to a test device to conduct a test for biomolecules using a microfluidic device and a control method thereof.

Microfluidic devices are used to perform biological or chemical reactions by manipulating small amounts of fluid.

A microfluidic structure provided in a microfluidic device to perform an independent function generally includes a chamber to accommodate a fluid, a channel allowing the fluid to flow therethrough, and a member (e.g., valve) to regulate the flow of the fluid. The chamber, channel, and valve may be disposed in the platform in a variety of configurations. The microfluidic structure may include various combinations of the chamber, channel, and valve. A device fabricated by disposing such a microfluidic structure on a chip-shaped substrate to perform a test involving an immune serum reaction or biochemical reaction on a small chip through multi-step processing and manipulation, is referred to as a "lab-on-a chip."

To transfer a fluid in a microfluidic structure, driving pressure is needed. Capillary pressure or pressure generated by a separate pump may be used as the driving pressure. Recently, a disk type microfluidic device which has a microfluidic structure arranged on a disk-shaped platform to move a fluid using centrifugal force to perform a series of operations has been proposed. This device is referred to as "Lab CD" or "Lab-on a CD."

A microfluidic device may include a chamber to detect an analyte or test material and a detection object such as test paper.

A test device is an apparatus provided with a light emitting element and a light receiving element to detect a detection object of the microfluidic device and thus detects results of biochemical reactions occurring within the detection object.

Depending on the condition, type, and color of the test paper, the brightness of an image of the detection object captured by the light receiving element may vary. This may lead to variations in assay results and reduce the reliability of such assay results.

Therefore, uniform brightness is needed regardless of the condition of the detection object when capturing an image of the detection in order to reduce variation in assay results and thus increase reliability of the assay results.

Exemplary embodiments provide a test device in which an image of a detection object may be obtained while maintaining uniform brightness thereof by control of an exposure level of a light receiving element through comparison of a signal value of the image of the detection object with a predetermined target value, and a control method thereof.

In accordance with an aspect of an exemplary embodiment, there is provided a test device to detect a detection object of a microfluidic device including: a light emitting element configured to emit light onto the microfluidic device, a light receiving element configured to capture an image of the detection object through light emitted from the light emitting element, and a controller configured to determine a signal value for a predetermined area of the image of the detection object captured by the light receiving element and adjust an exposure level of the light receiving element according to a difference between the signal value and a predetermined target value.

If the signal value is greater than the predetermined target value, the controller may decrease the exposure level of the light receiving element.

If the signal value is less than the predetermined target value, the controller may increase the exposure level of the light receiving element.

The controller may adjust the exposure level of the light receiving element by controlling at least one of shutter speed and iris opening of the light receiving element.

The detection object may include a test paper upon which a predetermined biochemical reaction of a biomaterial is performed, wherein the controller may determine a signal value for at least one predetermined area on an image of the test paper captured by the light receiving element.

The signal value may include an RGB signal value, a YCbCr signal value, or a Gray-scale signal value.

If the signal value is greater than the predetermined target value, and a difference between the signal value and the predetermined target value is greater than or equal to a predetermined standard value, the controller may reduce light emitted from the light emitting element such that the difference decreases below the standard value, and when the difference is less than the standard value, the controller may decrease the exposure level of the light receiving element.

If the signal value is less than the predetermined target value, and a difference between the signal value and the predetermined target value is greater than or equal to a predetermined standard value, the controller may increase light emitted from the light emitting element such that the difference decreases below the standard value, and when the difference is less than the standard value, the controller may increase the exposure level of the light receiving element.

The light emitting element may include a backlight unit as a surface light source.

The light receiving element may include a charge-coupled device (CCD) image sensor or a complementary metal-oxide-semiconductor (CMOS) image sensor.

In accordance with an aspect of another exemplary embodiment, there is provided a control method of a test device, the control method including: capturing a first image of a detection object of a microfluidic device using a light receiving element, determining a signal value for a predetermined area on the captured image of the detection object, adjusting an exposure level of the light receiving element in accordance with a difference between the signal value and a predetermined target value, and capturing a second image of the detection object.

The adjusting may include, if the signal value is greater than the predetermined target value, decreasing the exposure level of the light receiving element and capturing an image of the detection object.

The adjusting may include, if the signal value is less than the predetermined target value, increasing the exposure level of the light receiving element and capturing an image of the detection object.

The exposure level of the light receiving element may be adjusted by controlling at least one of shutter speed and iris opening of the light receiving element.

The detection object may include a test paper upon which a predetermined biochemical reaction of a biomaterial is performed, wherein the determining may include determining a signal value for at least one predetermined area on a captured image of the test paper.

The signal value may include an RGB signal value, a YCbCr signal value or a Gray-scale signal value.

The adjusting may include, if the signal value is greater than the predetermined target value and a difference between the signal value and the predetermined target value is greater than or equal to a predetermined standard value, reducing light emitted from the light emitting element such that the difference decreases below the standard value, and when the difference is less than the standard value, decreasing the exposure level of the light receiving element.

The adjusting may include, if the signal value is less than the predetermined target value and a difference between the signal value and the predetermined target value is greater than or equal to a predetermined standard value, increasing light emitted from the light emitting element such that the difference decreases below the standard value, and once the difference is less than the standard value, increasing the exposure level of the light receiving element.

### BRIEF DESCRIPTION OF THE DRAWINGS

The above and/or other aspects will become apparent and more readily appreciated from the following description of exemplary embodiments, taken in conjunction with the accompanying drawings of which:
FIG. 1 is a perspective view schematically illustrating a structure of a microfluidic device according to an exemplary embodiment;
FIGS. 2A to 2C are plan views illustrating the structure of the microfluidic device according to the exemplary embodiment in detail;
FIG. 3 is a graph schematically illustrating the rotational speed of a platform during respective fluid transferring operations in the microfluidic device according to the exemplary embodiment;
FIGS. 4A to 4E are plan views illustrating flow of a fluid in the microfluidic device according to the exemplary embodiment;
FIG. 5 is a function block diagram of the test device according to an exemplary embodiment;
FIG. 6 is a plan view illustrating a tray according to an exemplary embodiment, which is in an open state;
FIG. 7 is a plan view illustrating the tray according to the exemplary embodiment, which is in a closed state;
FIG. 8 is a plan view illustrating a bottom surface of an upper housing according to an exemplary embodiment;
FIG. 9 is a view illustrating the microfluidic device according to an exemplary embodiment, which is abnormally coupled to a rotary drive unit and a clamper;
FIG. 10 is a view illustrating the microfluidic device according to an exemplary embodiment, which is separated from the rotary drive unit and the clamper;
FIG. 11 is a view illustrating the microfluidic device according to an exemplary embodiment, which is normally coupled to a rotary drive unit and a clamper;
FIG. 12 is a flowchart illustrating a control method of a test device according to an exemplary embodiment;
FIG. 13 is a flowchart illustrating a control method of a test device according to another embodiment;
FIG. 14 is a flowchart showing a control method of a test device according to another embodiment;
FIG. 15 is a block diagram showing the configuration of a test device according to an exemplary embodiment;
FIG. 16 is a conceptual side view illustrating the configuration of the test device according to the an exemplary embodiment;
FIGS. 17 and 18 are views illustrating radial movement of a detection module according to an exemplary embodiment, which is seen from the top;
FIGS. 19 to 21 are views illustrating movement of the detection object to a position facing the light receiving element of the detection module by movement of the detection module and rotation of the microfluidic device in the test device according to the an exemplary embodiment, which is viewed from the top;
FIG. 22 is a block diagram illustrating a configuration of a test device according to another exemplary embodiment;
FIG. 23 is a conceptual side view illustrating the configuration of a test device according to another exemplary embodiment;
FIGS. 24 and 25 are views illustrating radial movement of a detection module according to another exemplary embodiment;
FIGS. 26 to 28 are views illustrating movement of a detection object to a position facing a light receiving element of a detection module by movement of the detection module and rotation of the microfluidic device in a test device according to another exemplary embodiment;
FIG. 29 is a flowchart illustrating a control method of a test device according to an exemplary embodiment;
FIG. 30 is a flowchart illustrating a control method of a test device according to another exemplary embodiment;
FIG. 31 is a view schematically illustrating test paper used as an example of the detection object of a test device according to an exemplary embodiment; and
FIG. 32 is a flowchart illustrating a control method of a test device according to an exemplary embodiment to capture an image of a detection object maintaining a uniform brightness of the test paper.

### DETAILED DESCRIPTION

Reference will now be made in detail to exemplary embodiments, examples of which are illustrated in the accompanying drawings, wherein like reference numerals refer to like elements throughout.

A microfluidic device according to an exemplary embodiment will be first described, and then a description will be given of a test device, such as a blood testing device, to detect a result of a biochemical reaction occurring on a detection object of the microfluidic device.

For the test device, a structure and control method of seating the microfluidic device in the test device will be first described and a detection module to detect the detection object positioned at various radii on the microfluidic device will be described, and finally a description will be given of a method of detecting the detection object while maintaining uniform brightness of the detection object to reduce variation in detection results of the detection object.

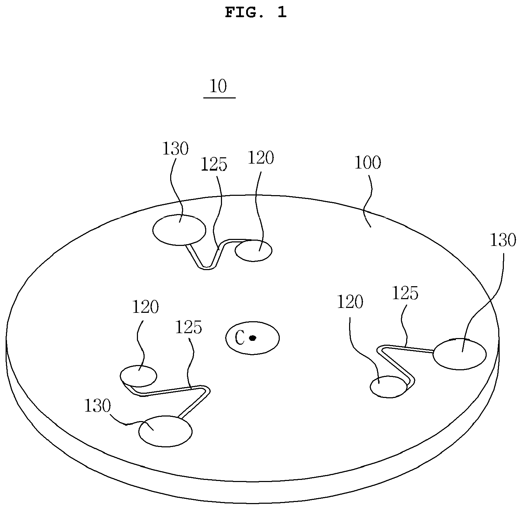

A microfluidic device according to an exemplary embodiment will be described below with reference to FIGS. 1 to 4.

FIG. 1 is a perspective view schematically illustrating a microfluidic device according to an exemplary embodiment.

Referring to FIG. 1, the microfluidic device 10 according to the illustrated embodiment includes a platform 100 on or within which one or more microfluidic structures are formed. The microfluidic structure includes a plurality of chambers to accommodate a fluid and a channel to connect the chambers.

Here, the microfluidic structure is not limited to a structure with a specific shape, but comprehensively refers to structures, including the channel connecting the chambers to each other, formed on or within the microfluidic device, especially on the platform of the microfluidic device to allow the flow of fluid. The microfluidic structure may perform different functions, depending on the arrangements of the chambers and the channels, and the kind of the fluid accommodated in the chambers or flowing within the channels.

The platform 100 may be made of various materials including plastic materials such as polymethylmethacrylate (PMMA), polydimethylsiloxane (PDMS), polycarbonate (PC), polypropylene, polyvinyl alcohol and polyethylene, glass, mica, silica and silicon wafer, which are easy to work with and whose surfaces are biologically inactive. The above materials are simply examples of materials usable for the platform 100, and the exemplary embodiments described herein are not limited thereto. Any material having proper chemical and biological stability, optical transparency and mechanical workability may be used as a material of the platform 100.

The platform 100 may be formed in multiple layers of plates. A space to accommodate a fluid within the platform 100 and a channel allowing the fluid to flow therethrough may be provided by forming intaglio structures corresponding to the microfluidic structures, such as the chambers and the channel, on the contact surfaces of two plates and thereafter, joining the plates. Joining two plates may be implemented using various techniques such as binding with an adhesive agent or a double-sided adhesive tape, ultrasonic welding, and laser welding.

The illustrated exemplary embodiment of FIG. 1 employs a circular plate-shaped disk type platform 100, but the platform 100 used in the illustrated embodiment may have the shape of a whole circular plate which is rotatable, may be a sector that is rotatable in a rotatable frame when seated thereon, or it may have any polygonal shape, provided that it is rotatable.

A platform 100 may be provided with one test unit. However, in order to expedite testing and cost efficiency, the platform 100 may be divided into a plurality of sections, and each section may be provided with a microfluidic structure which is operable independently of the other microfluidic structures. The microfluidic structures may be assigned to different tests and may perform several tests at the same time. Alternatively, a plurality of test units that perform the same test may be provided. For convenience of description of the illustrated exemplary embodiment, it will be hereinafter be assumed that a chamber to receive a sample from a sample supply chamber and a channel connected to the chamber form a single unit. Thus, if the sample supply chamber is different, the corresponding unit will be taken as a different unit.

Since the microfluidic device 10 according to the illustrated exemplary embodiment causes a fluid to move using centrifugal force, the chamber 130 to receive the fluid is disposed at a position more distant from the center C of the platform 100 than the position of the chamber 120 to supply the fluid, as shown in FIG. 1.

The two chambers are connected by the channel 125, and in the microfluidic device 10 of the illustrated embodiment, a siphon channel may be used as the channel 125 to control the fluid flowing therethrough.

As used herein, the term "siphon" refers to a channel that causes a fluid to move using a pressure difference. In the microfluidic device 10, the flow of the fluid through the siphon channel is controlled using capillary pressure that forces the fluid to move up through a tube having a very small cross-sectional area, and centrifugal force generated by rotation of the platform 100. That is, the inlet of the siphon channel, which has a very small cross-sectional area is connected to the chamber 120 in which the fluid is accommodated, and the outlet of the siphon channel is connected to another chamber 130to which the fluid is transferred. As shown, the bend in the siphon channel, i.e., the crest point of the siphon channel should be higher than the level of the fluid accommodated in the chamber 120. Also, since the fluid positioned closer to the outer edge of the platform 100 than the inlet of the siphon channel is not transferred, the positioning of the inlet of the siphon channel will depend on the amount of the fluid to be transferred. When the siphon channel is filled with the fluid by the capillary pressure of the siphon channel, the fluid filling the siphon channel is transferred to the next chamber by centrifugal force.

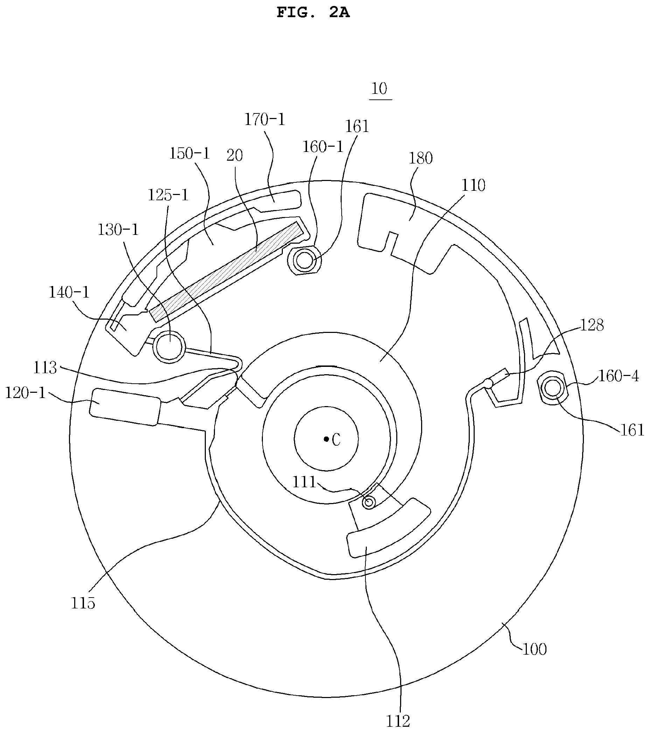

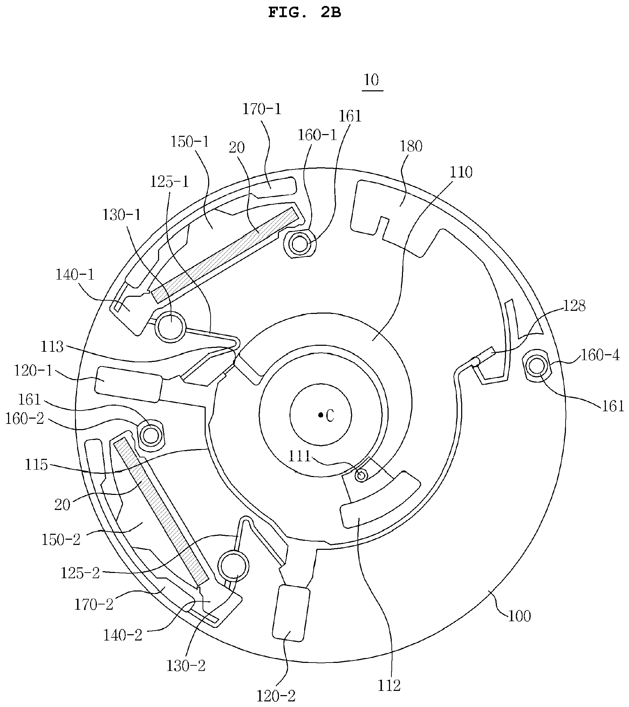

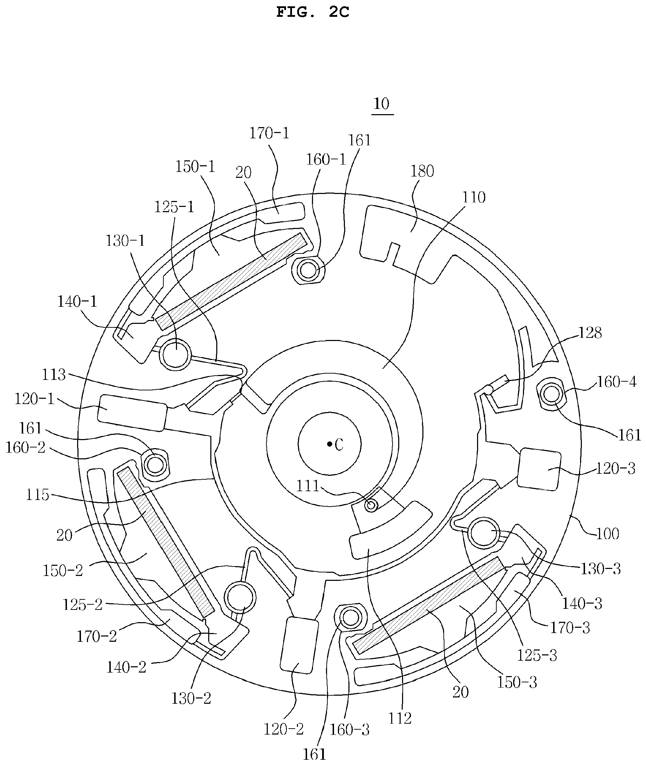

Hereinafter, the structure and operation of the microfluidic device according to the illustrated exemplary embodiment will be described in detail with reference to FIGS. 2 to 4.

FIG. 2 is a plan view specifically illustrating the structure of the microfluidic device according to the exemplary embodiment. Hereinafter, the structure of the microfluidic device 10 will be described in detail with reference to FIG. 2.

As described above, the platform 100 may be formed in various shapes including circle, sector and polygon, and in the illustrated embodiment, the platform 100 has a circular shape. Also, as shown in FIGS. 2A to 2C, at least one of first chambers may be connected to a distribution channel. For convenience of description, in the illustrated embodiment, it will be assumed that three first chambers 120, namely, chambers 120-1, 120-2 and 120-3 are connected in parallel to the distribution channel 115 and three second chambers 130-1,130-2 and 130-3 are connected to the respective first chambers 120, as shown in FIG. 2C.

The sample supply chamber 110 is formed at a position close to the center of rotation C to accommodate a sample supplied from the outside. The sample supply chamber 110 accommodates a fluidic sample, such as blood.

A sample introduction inlet 111 is provided at one side of the sample supply chamber 110, through which an instrument such as a pipette may be used to introduce blood into the sample supply chamber 110. Blood may be spilled near the sample introduction inlet 111 during the introduction of blood, or the blood may flow backward through the sample introduction inlet 111 during rotation of the platform 100. To prevent the microfluidic device 10 from being contaminated by such events, a backflow receiving chamber 112 may be formed on the upper surface of the microfluidic device 10 adjacent to the sample introduction inlet 111 to accommodate any spilled sample during the introduction thereof or any sample that flows backward.

In another exemplary embodiment, to prevent backflow of the blood introduced into the sample supply chamber 110, a structure that functions as a capillary valve may be formed in the sample supply chamber 110. Such a capillary valve allows passage of the sample only when a pressure greater than or equal to a predetermined level is applied.

In another exemplary embodiment, to prevent backflow of the blood introduced into the sample supply chamber 110, a rib-shaped backflow prevention device may be formed in the sample supply chamber 110. Such a rib-shaped back flow prevention device may include one or more protrusions formed on a surface of the sample supply chamber 110. Arranging the backflow prevention device in a direction crossing the direction of flow of the sample from the sample introduction inlet 111 to the sample discharge outlet may produce resistance to flow of the sample, thereby preventing the sample from flowing toward the sample introduction inlet 111.

The sample supply chamber 110 may be formed to have a width that gradually increases from the sample introduction inlet 111 to the sample discharge outlet 113 in order to facilitate discharge of the sample accommodated therein through the sample discharge outlet 113. In other words, the radius of curvature of at least one side wall of the sample supply chamber 110 may gradually increase from the sample introduction inlet 111 to the sample discharge outlet 113.

The sample discharge outlet 113 of the sample supply chamber 110 is connected to a distribution channel 115 formed on the platform 100 in the circumferential direction of the platform 100. Thus, the distribution channel 115 is sequentially connected to the "1-1"th chamber 120-1, the "1-2"th chamber 120-2 and the "1-3"th chamber 120-3 as it extends counterclockwise. A Quality Control (QC) chamber 128 to indicate completion of supply of the sample and an excess chamber 180 to accommodate any excess of the sample remaining after supply of the sample may be connected to the end of the distribution channel 115.

The first chambers 120 (i.e., 120-1, 120-2, and 120-3) may accommodate the sample supplied from the sample supply chamber 110 and cause the sample to separate into supernatant and sediment through centrifugal force. Since the exemplary sample used in the illustrated embodiment is blood, the blood may separate into a supernatant including serum and plasma, and sediment including corpuscles in the first chambers 120.

Each of the first chambers 120-1, 120-2 and 120-3 is connected to a corresponding siphon channel 125-1,125-2 and 125-3. As described above, the crest points (i.e., bend) of the siphon channels 125 should be higher than the highest level of the fluid accommodated in the first chambers 120. To secure a difference in height, the "1-2"th chamber 120-2 is positioned on a circumference that is further from the center of rotation C, or a circumference of a larger radius, than the circumference on which the "1-1"th chamber 120-1 is positioned, and the "1-3"th chamber 120-3 is positioned on a circumference that is further from the center of rotation C, or a circumference of a larger radius, than the circumference on which the "1-2"th chamber 120-2 is positioned.

In this arrangement, a chamber 120 positioned farther away from the sample discharge outlet 113 along the direction of flow of the distribution channel 115, will have a shorter overall length in a radial direction. Accordingly, if the first chambers 120 are set to have the same volume, then each of the successive first chambers 120 positioned farther away from the sample discharge outlet 113 may have a larger width in a circumferential direction, as shown in FIG. 2.

As described above, the positions at which the inlets of the siphon channels 125-1,125-2 and 125-3 meet the outlets of the first chambers 120-1, 120-2 and 120-3 may vary depending on the amount of fluid to be transferred. Thus, when the sample is blood, as in the illustrated exemplary embodiment, a test is often performed only on the supernatant, and therefore the outlets of the first chambers 120 may be arranged at upper portions (i.e., above the middle portion) thereof, at which the supernatant is positioned. Also, one or more protrusions connected to the siphon channels 125-1, 125-2 and 125-3 may be provided at the outlets of the first chambers 120 to facilitate flow of the fluid. However, it should be understood that when the sample is not blood or a test is to be performed on the sediment in addition to the supernatant, outlets may be provided at lower portions of the first chambers 120.

The outlets of the siphon channels 125-1,125-2 and 125-3 are connected to the respective second chambers 130-1,130-2 and 130-3. The second chambers 130 may accommodate only sample (e.g., blood), or may have a reagent or reactant pre-stored therein. The reagent or reactant may be used, for example, to perform a pretreatment or first order reaction for blood, or to perform a simple test prior to the main test. In the illustrated exemplary embodiment, binding between an analyte and a first marker conjugate occurs in at least one of the second chambers 130-1,130-2 and 130-3.

Specifically, the first marker conjugate may remain in the second chamber 130 in a liquid phase or solid phase. When the marker conjugate remains in a solid phase, the inner wall of the second chamber 130 may be coated with the marker conjugate or the marker conjugate may be temporarily immobilized on a porous pad disposed therein.

The first marker conjugate is a complex formed by combining a marker and a capture material which specifically reacts with an analyte in the sample. For example, if the analyte is antigen Q, the first marker conjugate may be a conjugate of the marker and antibody Q which specifically reacts with antigen Q.

Exemplary markers include, but are not limited to, latex beads, metal colloids including gold colloid and silver colloid, enzymes including peroxidase, fluorescent materials, luminous materials, superparamagnetic materials, materials containing lanthanum (III) chelate, and radioactive isotopes.

Also, if test paper on which a chromatographic reaction occurs is inserted into the reaction chamber 150, as described below, a second marker conjugate which binds with a second capture material may be immobilized on the control line of the test paper to confirm reliability of the reaction. In various exemplary embodiments, the second marker conjugate may also be in a liquid phase or solid phase, and when it is in a solid phase, the inner wall of the second chamber 130 may be coated with the second marker conjugate or the second marker conjugate may be temporarily immobilized on a porous pad disposed therein.

The second marker conjugate is a conjugate of the marker and a material specifically reacting with the second capture material immobilized on the control line. The marker may be one of the aforementioned exemplary materials. If the second capture material immobilized on the control line is biotin, a conjugate of streptavidin and the marker may be temporarily immobilized in the second chamber 130.

Accordingly, when blood flows into the second chamber 130, antigen Q present in the blood is discharged to the third chamber 140 along with the flow of blood, binding with the first marker conjugate having antibody Q. At this time, the second marker conjugate having streptavidin is also discharged.

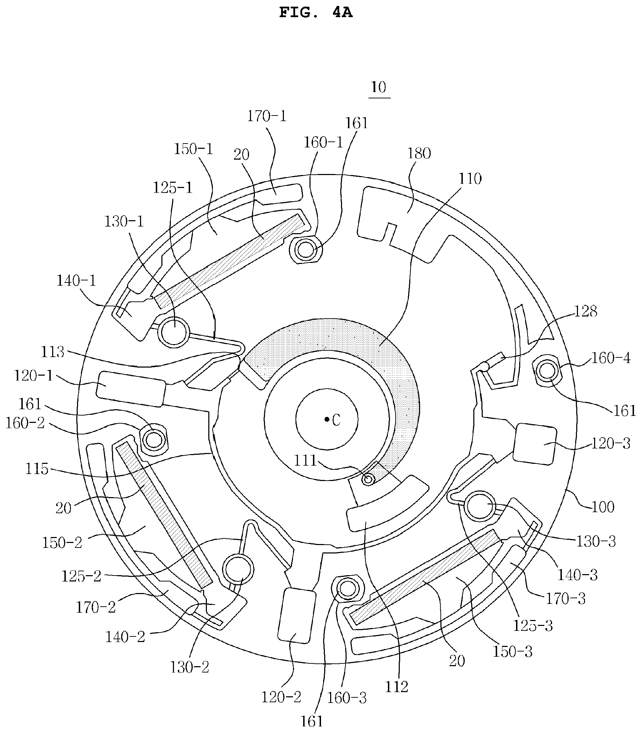

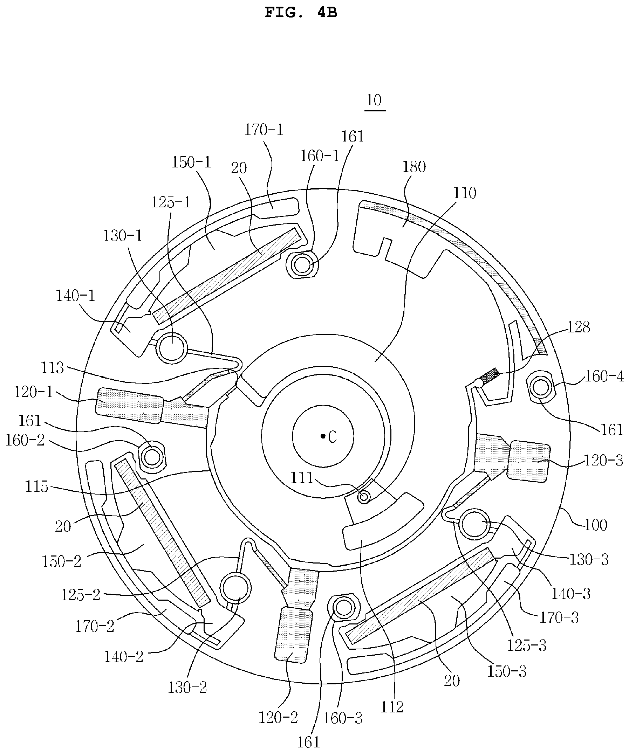

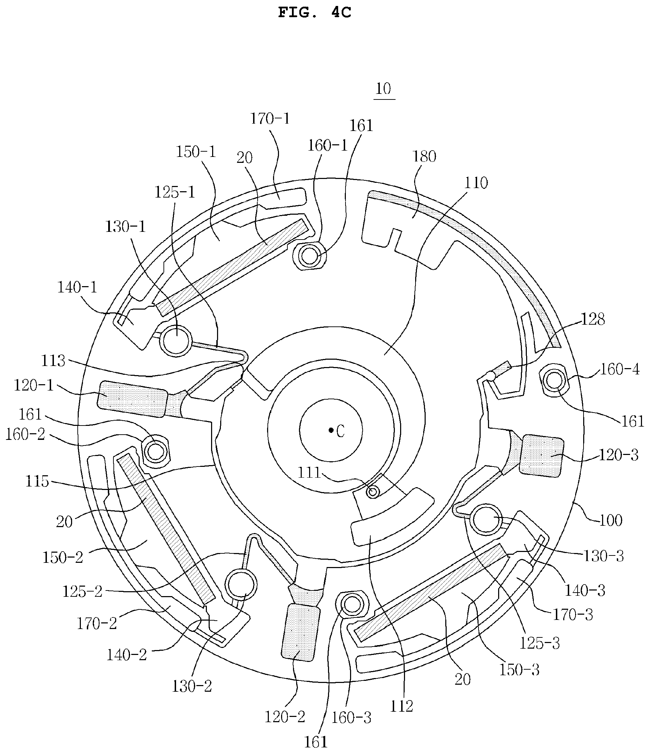

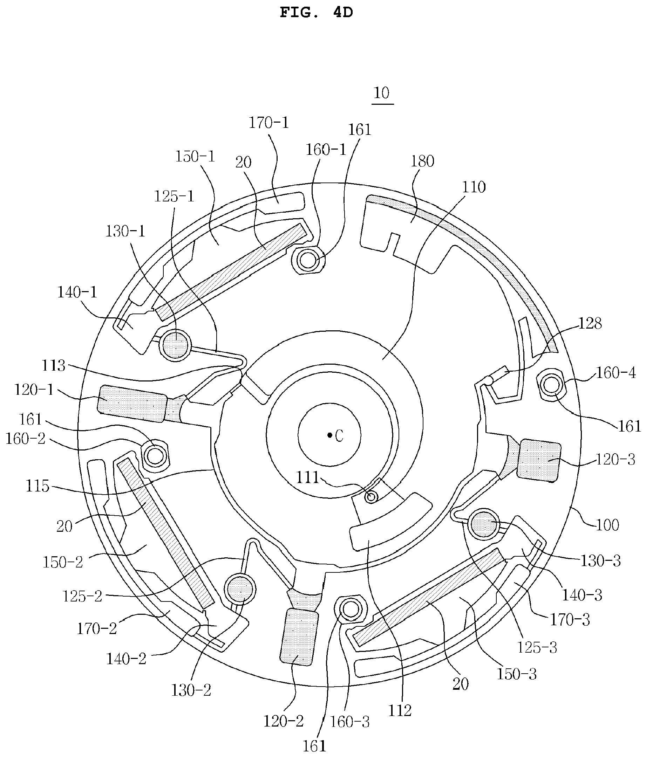

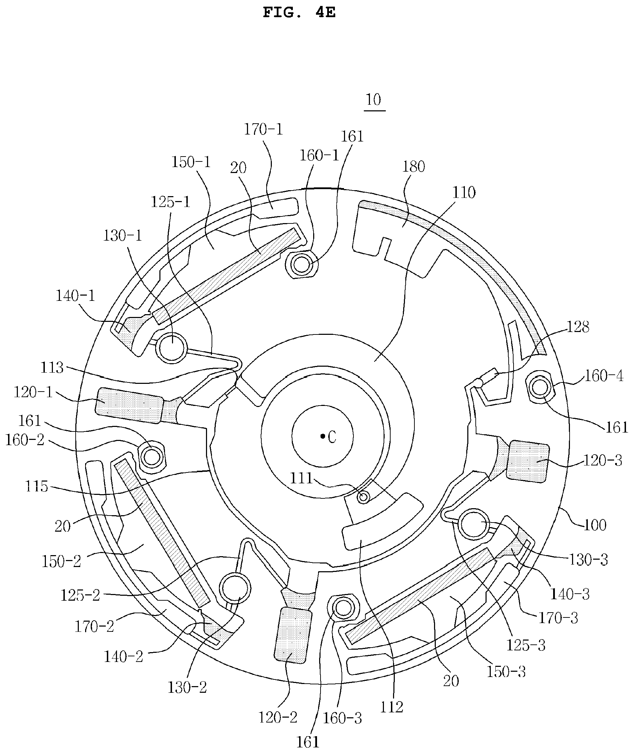

The second chambers 130-1,130-2 and 130-3 are connected to the respective third chambers 140-1,140-2 and 140-3, and in the illustrated exemplary embodiment, the third chambers 140 are used as metering chambers. The metering chambers 140 function to meter a fixed amount of sample (e.g., blood) accommodated in the second chamber 130 and supply the fixed amount of blood to the respective fourth chambers 150 (150-1, 150-2, and 150-3). The metering operation of the metering chambers will be described below with reference to FIG. 4.

The residue in the metering chambers 140 which has not been supplied to the fourth chambers 150 is transferred to the respective waste chambers 170 (170-1, 170-2, and 170-3).

The third chambers 140-1,140-2 and 140-3 are connected to the reaction chambers 150-1,150-2 and 150-3 which are the fourth chambers. A reaction may occur in the reaction chambers 150 in various ways. For example, in the illustrated embodiment, chromatography based on capillary pressure is used in at least one of the reaction chambers 150-1,150-2 and 150-3. To this end, the reaction chamber 150 includes a test paper-type detection object 20 to detect the presence of an analyte through chromatography.

The reaction chambers 150-1, 150-2 and 150-3 are connected to the respective fifth chambers, i.e., the waste chambers 170-1, 170-2 and 170-3. The waste chambers 170-1, 170-2 and 170-3 accommodate impurities wasted from the reaction chambers 150-1, 150-2 and 150-3.

In addition to chambers in which a sample or residue is accommodated or a reaction occurs, the platform 100 may be provided with one or more magnetic bodies disposed within magnetic body accommodating chambers 160-1,160-2,160-3 and 160-4 for position identification. The magnetic body accommodating chambers 160-1,160-2,160-3 and 160-4 accommodate a magnetic material, which may be formed of a ferromagnetic material such as iron, cobalt and nickel which have a high intensity of magnetization and are likely to form a strong magnet like a permanent magnet, or a paramagnetic material such as chromium, platinum, manganese, and aluminum which have a low intensity of magnetization and thus do not form a magnet by themselves, but may become magnetized when a magnet approaches to increase the intensity of magnetization thereof.

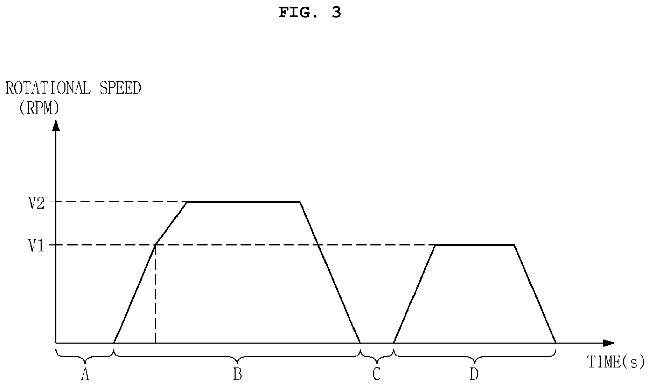

FIG. 3 is a graph schematically illustrating the rotational speed of the platform 100 during respective fluid transferring operations in the microfluidic device according to an exemplary embodiment, and FIGS. 4A to 4E are plan views illustrating flow of a fluid in the microfluidic device according to the exemplary embodiment. The microfluidic device of FIG. 4 has the same structure as the microfluidic device shown in FIG. 2.

Referring to FIG. 3, the operation of transferring the fluid in the microfluidic device 10 may be broadly divided into: introducing a sample (A), distributing the sample (B), wetting a siphon channel (C), and transferring the sample (D). Here, wetting refers to an operation of allowing the fluid to fill the siphon channel 125. Hereinafter, operations of the microfluidic device will be described by matching the graph of FIG. 3 to the plan views of FIG. 4 showing the respective operations.

FIG. 4A is a plan view of the microfluidic device 10 which is in the operation of introducing a sample (A). A sample is introduced into the sample supply chamber 110 through the sample introduction inlet 111 while the platform 100 is at rest (rpm=0). In the present exemplary embodiment, a blood sample is introduced. Since a backflow receiving chamber 112 is arranged at a portion adjacent to the sample introduction inlet 111, contamination of the microfluidic device 10 due to blood dropped on a place other than the sample introduction inlet 111 may be prevented during the operation of introducing the sample.

FIG. 4B is a plan view of the microfluidic device which is in the operation of distributing the sample (B). When introduction of the sample is completed, distribution of the sample to the first chambers 120 is initiated. At this time, the platform 100 begins to rotate and the rotational speed (rpm) thereof increases. If a test is performed on a blood sample as in the illustrated exemplary embodiment, centrifugation may be performed along with distribution of the sample. Through the centrifugation, the blood may separate into the supernatant and the sediment. The supernatant includes serum and plasma, and the sediment includes corpuscles. The portion of the sample substantially used in the test described herein is the supernatant.

As illustrated in FIG. 3, the rotational speed is increased to v1 to distribute the blood accommodated in the sample supply chamber 110 to the "1-1"th chamber 120-1, the "1-2"th chamber 120-2 and the "1-3"th chamber 120-3 using centrifugal force. Thereafter, the rotational speed is increased to v2 to allow centrifugation to occur within each chamber. When centrifugation occurs for the blood accommodated in each chamber, the supernatant gathers at a position proximal to the center of rotation, while the sediment gathers at a position distal to the center of rotation. In the exemplary embodiment shown in FIG. 4, the first chambers 120 are formed to contain the same volume of sample. However, the first chambers 120 may be formed in different sizes, depending on the amounts of fluid to be distributed thereto.

When the supply of blood to the "1-1"th chamber 120-1, the "1-2"th chamber 120-2 and the "1-3"th chamber 120-3 is completed, any excess blood not supplied to the first chambers 120 flows into the QC chamber 128 through the distribution channel 115. Further, any excess blood which does not flow into the QC chamber 128 flows into the excess chamber 180.

As shown in FIG. 4B, a magnetic body accommodating chamber 160-4 is formed at a position adjacent to the QC chamber 128. As such, a magnet within a detection module may cause the QC chamber 128 to face a light receiving element 1411 of the detection module, as discussed below. Accordingly, when the light receiving element 1411 faces the QC chamber 128, it may measure transmittance of the QC chamber 128 and determine whether the supply of the blood to the first chambers 120 has been completed.

FIG. 4C is a plan view of the microfluidic device which is in the operation of wetting a siphon channel (C). When distribution and centrifugation of the blood is completed, the platform 100 is stopped (rpm=0), thereby permitting the blood accommodated in the first chambers 120-1, 120-2 and 120-3 to fill the siphon channels 125-1,125-2 and 125-3 by capillary pressure.

FIG. 4D is a plan view of the microfluidic device which is in the operation of transferring the sample to the second chamber 130 (D). When wetting of the siphon channel 125 is completed, the platform 100 is rotated again to allow the blood filling the siphon channels 125-1,125-2 and 125-3 to flow into the second chambers 130-1,130-2 and 130-3. As shown in FIG. 4D, since the inlets of the siphon channels 125-1,125-2 and 125-3 are connected to the upper portions of the first chambers 120-1, 120-2 and 120-3 (the portions proximal to the center of rotation), the supernatant of the blood sample flows into the second chambers 130-1,130-2 and 130-3 through the siphon channels 125-1,125-2 and 125-3.

The second chambers 130 may simply serve to temporarily accommodate the blood flowing therein, or allow, as described above, binding between a specific antigen in the blood and a marker conjugate pre-provided in the second chambers 130 to occur.

FIG. 4E is a plan view of the microfluidic device which is in the operation of transferring the sample to the metering chambers 140 (D). The blood flowing into the second chambers 130-1,130-2 and 130-3 are then introduced into the third chambers, i.e., the metering chambers 140-1,140-2 and 140-3 by centrifugal force. By centrifugal force, the metering chambers 140-1,140-2 and 140-3 are filled with blood from the lower portion of the second chambers 130, i.e., from the portion distal to the center of rotation. After the metering chambers 140-1,140-2 and 140-3 are filled with blood up to the outlets thereof, blood subsequently introduced into the metering chambers 140-1,140-2 and 140-3 flows into the reaction chambers 150-1,150-2 and 150-3 through the outlets of the metering chambers 140-1,140-2 and 140-3. Therefore, the positions of the outlets of the metering chambers 140 may be adjusted to supply a fixed amount of blood to the reaction chambers 150.

The reaction occurring in the reaction chambers 150 may be a reaction by immune chromatography or a binding reaction with a capture antigen or capture antibody, as described above.

As shown in FIG. 4E, if the magnetic body accommodating chambers 160-1, 160-2 and 160-3 are formed at positions adjacent to the corresponding reaction chambers 150-1,150-2 and 150-3, the positions of the reaction chambers 150-1,150-2 and 150-3 may be identified by a magnet within the detection module, as described below.

Hereinafter, a detailed description will be given of a test device according to an exemplary embodiment.

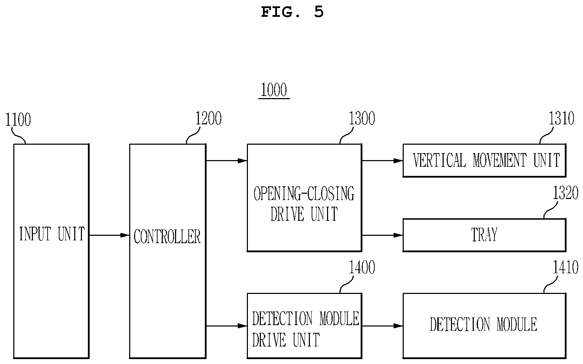

Constituents related to seating the microfluidic device for rotation will be described with reference to FIGS. 5 to 14.

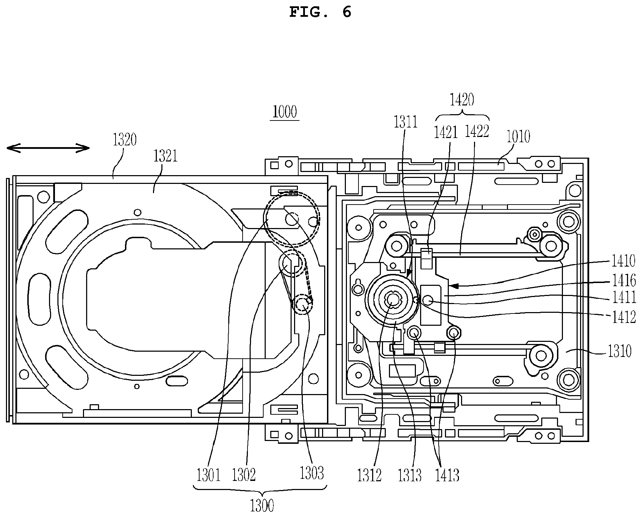

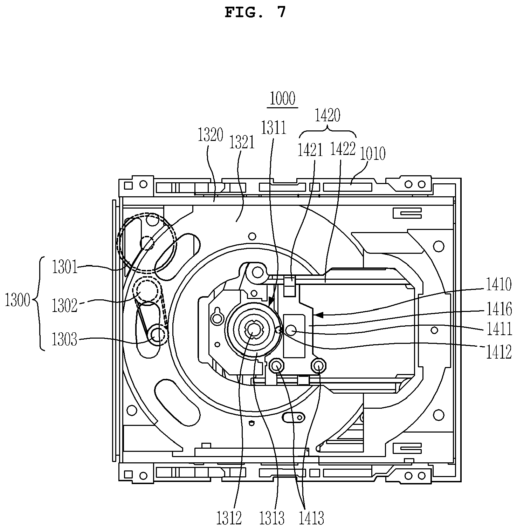

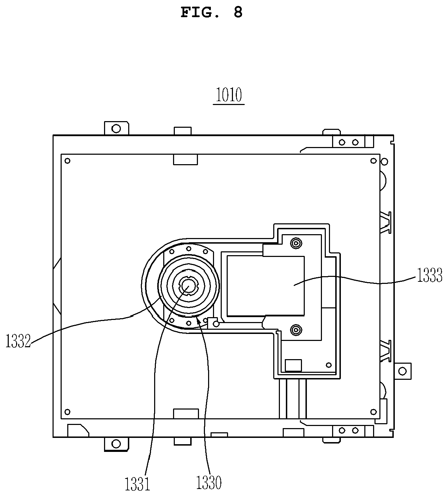

FIG. 5 is a function block diagram of the test device according to an exemplary embodiment, FIG. 6 is a plan view illustrating a tray according to the exemplary embodiment, which is in an open state, and FIG. 7 is a plan view illustrating the tray according to the exemplary embodiment, which is in a closed state, and FIG. 8 is a plan view illustrating the bottom surface of an upper housing according to the exemplary embodiment.

As shown in FIGS. 5 to 8, a test device 1000 includes an input unit 1100 allowing a user to input a command therethrough from the outside, a controller 1200 to control overall operation and functions of the test device 1000 according to a command from the input unit 1100, an opening-closing drive unit 1300 to drive a vertical movement part 1310 or a tray 1320 according to a command from the controller 1200, a detection module drive unit 1400 to drive a detection module 1410 according to a command from the controller 1200.

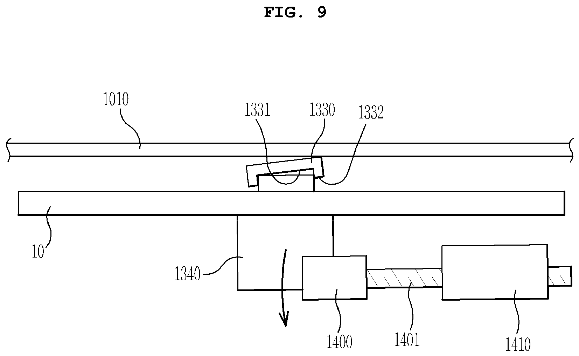

The controller 1200 controls operations related to the rotary drive unit 1340 (Figure 9) of the test device 1000, and controls operations of the tray 1320 and the vertical movement part 1310 to mount the microfluidic device 10 on the test device 1000. Also, the controller 1200 controls operation of the detection module 1410 to perform a function related to detection of the detection object 20 in the microfluidic device 10.

The rotary drive unit 1340 rotates the microfluidic device 10 using a spindle motor. The rotary drive unit 1340 may receive a signal output from the controller 1200 to repeat the operation of rotating and stopping, thereby generating centrifugal force to transfer the fluid within the microfluidic device 10 or to move various structures in the microfluidic device 10 to desired positions. Also, the rotary drive unit 1340 may include a motor drive to control the angular position of the microfluidic device 10. For example, the motor drive may employ a stepping motor or a direct current (DC) motor.

The microfluidic device 10 is seated on the tray 1320. The tray 1320 may be moved to a first state in which the tray 1320 is positioned outside the test device 1000 (i.e., opened) and to a second state in which the tray 1320 is positioned inside the test device 1000 (i.e., closed).

The vertical movement part 1310 may perform a first movement during which the vertical movement part 1310 moves to an upper side at which the tray 1320 is arranged and a second movement which is performed in the direction opposite to the first movement. Through movements of the vertical movement part 1310, the microfluidic device 10 may be correctly seated on the tray 1320. Also, by force acting between the clamper 1330 positioned at the housing 1010 and the rotary drive unit 1340, the clamper 1330 may press the microfluidic device 10. For example, the force acting between the clamper 1330 and the rotary drive unit 1340 may be magnetic force. Accordingly, the microfluidic device 10 may be seated at a set position on the test device 1000, and thereby errors in a test such as failure to perform the test due to failure to seat the microfluidic device 10 at the set position may be prevented. Also, since the microfluidic device 10 is pressed by the clamper 1330, displacement of the microfluidic device 10 from the tray 1320 may be prevented during operation of the test device 1000.

The microfluidic device 10 may be loaded onto the tray 1320, and microfluidic structures and a magnetic body 161 may be positioned in the microfluidic device 10. The detection object 20 is positioned in at least one of the microfluidic structures. For the magnetic body 161, a ferromagnetic material or paramagnetic material may be used. The detection object 20 may be assay site or test paper. The platform 100 of the microfluidic device 10 may be a non-optical bio disk or optical bio disk.

The detection module 1410 may include a light receiving element 1411. Also, the detection module 1410 may include at least one magnet 1413 to guide the microfluidic device 10 to the exact position thereof. In addition, the detection module 1410 may move within the housing 1010 of the test device 1000. The movement of the detection module 1410 performed in the direction in which the tray 1320 is accommodated in the housing 1010 is defined as a third movement, and the movement thereof performed in the direction opposite to the third movement is defined as a fourth movement. That is, when the microfluidic device 10 is seated on the tray 1320, the third movement and the fourth movement are performed in a radial direction relative to the microfluidic device 10. As such, through the movements of the detection module 1410, the light receiving element 1411 may be moved to perform detection at various positions. In addition, the microfluidic device 10 may be seated at a set position by attractive force created between the magnet 1413 within the detection module 1410 and the magnetic body 161 within the microfluidic device 10.

The test device 1000 includes the housing 1010 forming an external appearance thereof, the tray 1320 onto which the microfluidic device 10 is loaded, and the vertical movement part 1310 positioned inside the housing 1010 and coupled to the microfluidic device 10 to rotate the microfluidic device 10.

The housing 1010 may include an upper housing and a lower housing. In an exemplary embodiment, a light emitting element 1333 may be positioned at the upper housing, and a vertical movement part 1310 may be positioned at the lower housing. The vertical movement part 1310 may be arranged at the lower side of the tray 1320. The tray 1320 may be accommodated in the lower housing when in the second state (i.e., in the closed position). The detection module 1410 may be arranged at the vertical movement part 1310. As the detection module 1410 is provided with the light receiving element 1411, the light emitting element 1333 and the light receiving element 1411 may be arranged such that the microfluidic device 10 is placed therebetween.

The tray 1320 may be provided with a seating surface 1321 on which the microfluidic device 10 is seated. The seating surface 1321 is formed in a shape corresponding to that of the microfluidic device 10 to stably support the microfluidic device 10. The tray 1320 may be moved to the first state (i.e., opened position) and the second state (i.e., closed position) by an opening-closing drive unit 1300 positioned at the lower side of the tray 1320. The tray 1320 may be controlled by the controller 1200 to be opened or closed. Alternatively, the tray 1320 may be opened and closed by control of a dedicated OPEN/CLOSE button instead of the controller 1200.

The vertical movement part 1310 may be provided with a rotary drive unit 1340 which is inserted into a through hole of the microfluidic device 10 through an opening of the tray 1320. The rotary drive unit 1340 is coupled to the microfluidic device 10 to rotate the microfluidic device 10. A head portion 1311 coupled to the microfluidic device 10 is provided at the upper side of the rotary drive unit 1340. The head portion 1311 may be provided with an insert portion 1312 inserted into the through hole of the microfluidic device 10, and a support portion 1313 adapted to contact the lower surface of the microfluidic device 10 when the rotary drive unit 1340 is completely inserted into the through hole. When the microfluidic device 10 is mounted to the tray 1320, the rotary drive unit 1340 moves into position to be coupled in the through hole of the microfluidic device 10. After the rotary drive unit 1340 is coupled in the through hole, it rotates the microfluidic device 10.

The vertical movement part 1310 may be used to perform the first movement of moving toward the position of the tray 1320 by the opening-closing drive unit 1300, and the second movement which is made in the direction opposite to the first movement. Thus, the rotary drive unit 1340 is moved in accordance with the vertical movement part 1310. The vertical movement part 1310 may be moved by the opening-closing drive unit 1300 located at the lower side of the tray 1320.

The opening-closing drive unit 1300 includes a drive motor 1301. The drive motor 1301 is coupled to a gear (not shown), which is in turn, coupled to a drive pulley 1302. The drive pulley 1302 transmits power to a driven pulley 1303. The driven pulley 1303 is coupled to the tray 1320 such that the tray 1320 is moved to the first state and the second state. According to the illustrated embodiment, when the tray 1320 is moved to the second state, the vertical movement part 1310 automatically makes the first movement toward the tray 1320.

Mounted at one side of the vertical movement part 1310 is the detection module 1410. The detection module 1410 may include a component to detect the detection object 20 of the microfluidic device 10. The detection module 1410 may include a plate 1416 at which the component is positioned. The detection module 1410 may be slidably moved by a guide member 1420 provided at the vertical movement part 1310. The guide member 1420 includes one or more rod-shaped supports 1422 and one or more coupling portions 1421 protruding to the upper side of the plate 1416. The plate 1416 may be coupled to the support rods 1422 to move within the housing 1010 along the support rods 1422, from an inner circumference to an outer circumference relative to the rotary drive unit 1340. The coupling portion 1421 is slidably mounted to the support rod 1422 to support the detection module 1410 and at the same time to allow the detection module 1410 to move along the support rods 1422.

The upper housing 1010 is positioned at the upper side of the tray 1320. The upper housing 1010 is provided with the clamper 1330 to fix the microfluidic device 10 to the rotary drive unit 1340. The clamper 1330 may include a accommodation portion 1331 to accommodate the rotary drive unit 1340 and a protrusion 1332 protruding to form an outer circumference that protrudes toward the surface of the microfluidic device 10 when loaded onto the tray 1320, to be firmly coupled to the rotary drive unit 1340. The clamper 1330 may be arranged to make movements with respect to the upper housing 1010.

The accommodation portion 1331 of the clamper 1330 may be provided with a magnetic body, and a magnet may be provided at the upper portion of the rotary drive unit 1340. Alternatively, the accommodation portion 1331 of the clamper 1330 may be provided with a magnet, and a magnetic body may be provided at the upper portion of the rotary drive unit 1340.

When the through hole of the microfluidic device 10 loaded onto the tray 1320 is seated on the rotary drive unit 1340, the clamper 1330 presses the microfluidic device 10 using the magnetic force generated between the clamper 1330 and the rotary drive unit 1340. Thereby, shaking of the microfluidic device 10 may be prevented during operation thereof, and the through hole of the microfluidic device 10 may remain seated on the rotary drive unit 1340 even when attractive force acts between the magnet 1413 mounted to the detection module 1410 and the magnetic body 161 of the microfluidic device 10. To this end, the magnetic force between the clamper 1330 and the rotary drive unit 1430 is set to be stronger than the magnetic force between the magnetic body 161 mounted to the microfluidic device 10 and the magnet 1413 mounted to the detection module 1410.

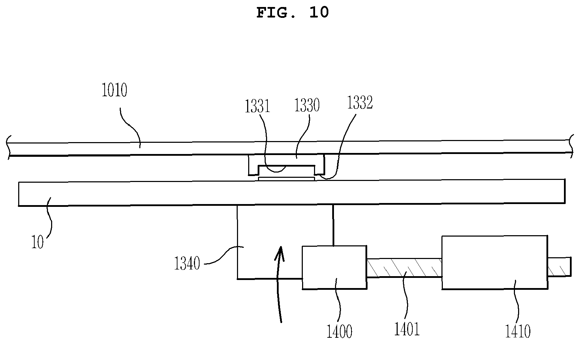

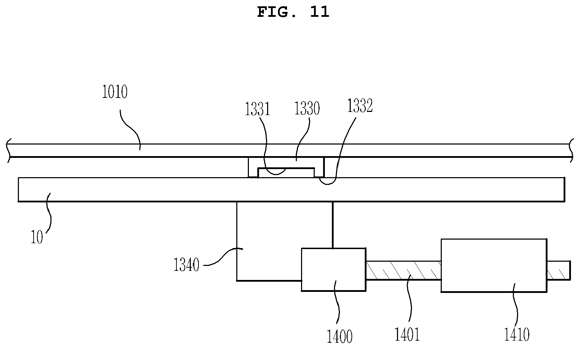

FIG. 9 is a view illustrating the microfluidic device 10 according to an exemplary embodiment, which is abnormally coupled to a rotary drive unit 1340 and a clamper 1330, FIG. 10 is a view illustrating the microfluidic device according to the exemplary embodiment, which is separated from the rotary drive unit and the clamper, and FIG. 11 is a view illustrating the microfluidic device according to the exemplary embodiment, which is coupled normally to a rotary drive unit and a clamper.

As shown in FIGS. 9 to 11, the rotary drive unit 1340 is vertically moveable. Independently of the rotary drive unit 1340, the detection module 1410 is moveably within the housing 1010.

The rotary drive unit 1340 is moved by the opening-closing drive unit 1300, while the detection module 1410 is moved by the detection module drive unit 1400.

The through hole of the microfluidic device 10 is seated on the rotary drive unit 1340. The rotary drive unit 1340 may perform the first movement and the second movement at least once. Accordingly, if the rotary drive unit 1340 is abnormally coupled to the clamper 1330 as shown in FIG. 9, the rotary drive unit 1340 may perform the second movement. By the second movement of the rotary drive unit 1340, the rotary drive unit 1340 and the clamper 1330 are separated from each other as shown in FIG. 10. Since separation of the microfluidic device 10 from the clamper 1330 causes the tray 1320 to be opened, the rotary drive unit 1340 makes the second movement only to the extent at which the rotary drive unit 1340 is not separated from the microfluidic device 10. Thereafter, when the rotary drive unit 1340 makes the first movement after the second movement, the rotary drive unit 1340 is coupled to the clamper 1330 through the microfluidic device 10 and the tray 1320, as shown in FIG. 11.

The rotary drive unit 1340 may be moved by movement of the vertical movement part 1310. Power produced by the detection module drive unit 1400 is transmitted to the detection module 1410 through the power transmission part 1401 to allow the detection module 1410 to move in a radial direction relative to the microfluidic device 10. Thereby, if the rotary drive unit 1340 is abnormally coupled to the clamper 1330, errors may be corrected without a separate manipulation by a user.

Typically, if the clamper 1330 arranged at the upper housing 1010 is abnormally seated between the microfluidic device 10 and the upper housing 1010, the microfluidic device 10 fails to rotate. In the illustrated exemplary embodiment, however, a gap is created between the rotary drive unit 1340 and the clamper 1330 by the second movement of the rotary drive unit 1340. As such, even when the clamper 1330 is abnormally seated, an error is corrected by allowing the clamper 1330 to be normally seated.

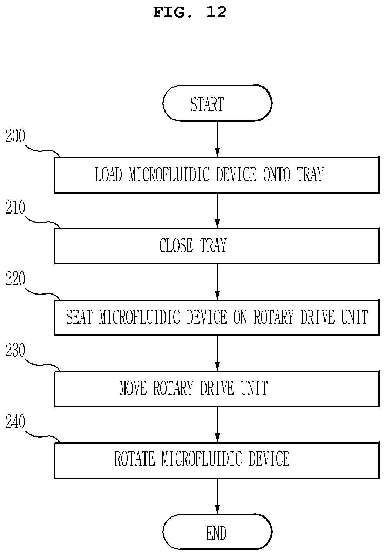

FIG. 12 is a flowchart illustrating a control method of a test device according to an exemplary embodiment.

As shown in FIG. 12, a control method of the test device 1000 includes loading the microfluidic device 10 onto the tray 1320 (200), seating the center of the microfluidic device 10 on the rotary drive unit 1340 (220), and making, by the rotary drive unit 1340, a vertical movement at least once (230).

First, the microfluidic device 10 is loaded onto the tray 1320 (200). The tray 1320 is moved to the second state in which it is accommodated in the housing 1010 and closed (210). When the tray 1320 is closed, the rotary drive unit 1340 is coupled to the through hole of the microfluidic device 10, thereby seating the microfluidic device 10 on the rotary drive unit 1340 (220). The rotary drive unit 1340 may move to enable normal coupling to the clamper 1330 such that the microfluidic device 10 is coupled to the clamper 1330 (230). According to the illustrated embodiment, even when the first movement of the rotary drive unit 1340 is performed once and allows the rotary drive unit 1340 to be coupled to the microfluidic device 10, the rotary drive unit 1340 may make the second movement and then repeat the first movement as needed. As such, the rotary drive unit 1340 may perform the first movement and the second movement at least once. Thus, when the clamper 1330 is incorrectly coupled to the rotary drive unit 1340 after the first movement of the rotary drive unit 1340, the position of the clamper 1330 may be corrected by performing the second movement of the rotary drive unit 1340 and then repeating the first movement again. When the rotary drive unit 1340 is coupled to the vertical movement part 1310, as in the illustrated exemplary embodiment, the vertical movement part 1310 may perform the first movement and the second movement to move the rotary drive unit 1340.

The controller 1200 controls the second movement made by the rotary drive unit 1340 and/or the vertical movement part 1310 such that the microfluidic device 10 is not separated from the rotary drive unit 1340 during the movement. This is because movement of the second movement of the rotary drive unit 1340 beyond a certain distance could cause the tray 1320 to be opened, as the vertical movement part 1310 and the tray 1320 are moved by the opening-closing drive unit 1300. Since the rotary drive unit 1340 needs to be moved by a distance that does not cause the tray 1320 to be opened, the rotary drive unit 1340 is moved to an extent that does not cause the microfluidic device 10 to be displaced from the rotary drive unit 1340. Once the microfluidic device 10 is seated normally on the rotary drive unit 1340 through the above operations, the microfluidic device 10 begins to rotate (240).

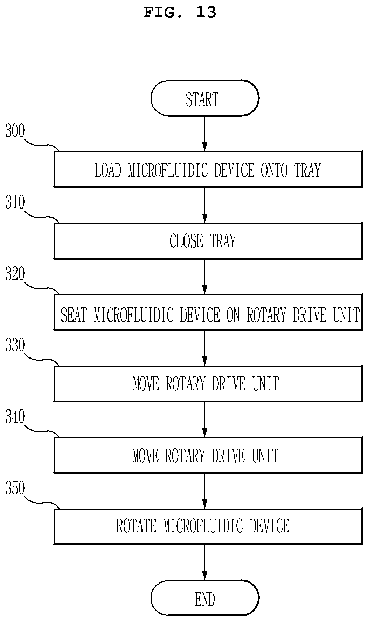

FIG. 13 is a flowchart illustrating a control method of a test device according to another exemplary embodiment.

According to the illustrated exemplary embodiment shown in FIG. 13, in addition to movement of the rotary drive unit 1340 (330), the detection module 1410 may also be moved (340). Compared to the rotary drive unit 1340, the detection module 1410 may make a third movement during which the detection module 1410 moves to one side of the housing 1010, and a fourth movement which is performed in the direction opposite to that of the third movement. Due to at least one magnet 1413 of the detection module 1410 and the magnetic body 161 of the microfluidic device 10, the detection module 1410 may be moved to cause the microfluidic device 10 to be stably seated. Through movement of the detection module 1410, fine adjustment of the position of the microfluidic device 10 is performed when the microfluidic device 10 is seated.

When the microfluidic device 10 is coupled to the clamper 1330, the detection module 1410 may be moved to an outermost position within the housing 1010 (i.e., beyond the outer circumference of the microfluidic device 10). This is intended to prevent the magnet 1413 from affecting rotation of the microfluidic device 10 during an assay using the microfluidic device 10.

In conventional cases, movement of the detection module 1410 has caused the microfluidic device 10 to move and interfere with normal rotation. However, in the illustrated exemplary embodiment, seating of the microfluidic device 10 is controlled not only by the movement of the detection module 1410, but also by the movement of the rotary drive unit 1340. Thus, operational errors resulting from movement of the detection module 1410 may be avoided.

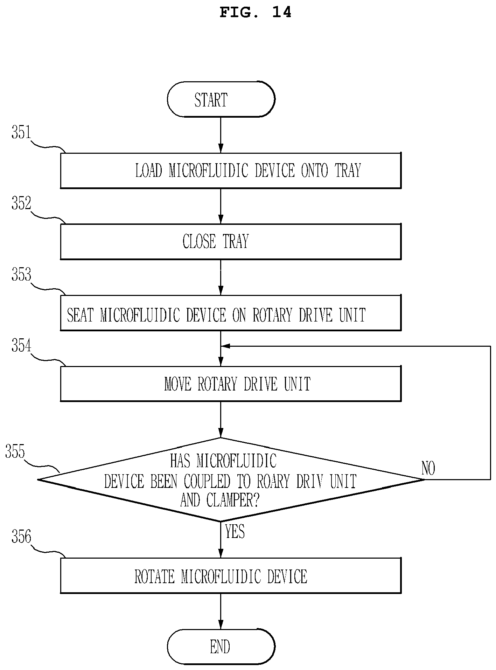

FIG. 14 is a flowchart showing a control method of a test device according to another exemplary embodiment.

In accordance with the illustrated exemplary embodiment shown in FIG. 14, after the rotary drive unit 1340 has moved, the controller 1200 may determine whether the microfluidic device 10 is properly coupled to the rotary drive unit 1340 and the clamper 1330 (355). For example, the coupling may be checked through the conditions of the rotary drive unit 1340 and the clamper 1330. If the rotary drive unit 1340 and the clamper 1330 are properly coupled, magnetic force is produced between the clamper 1330 and the rotary drive unit 1340. Based on this magnetic force, it may be determined whether the microfluidic device 10 is properly seated on the rotary drive unit 1340. If it is determined that the microfluidic device 10 is normally seated, the microfluidic device 10 begins to rotate (356). If the microfluidic device 10 is not properly seated, the first movement and the second movement of the rotary drive unit 1340 are performed again (354).

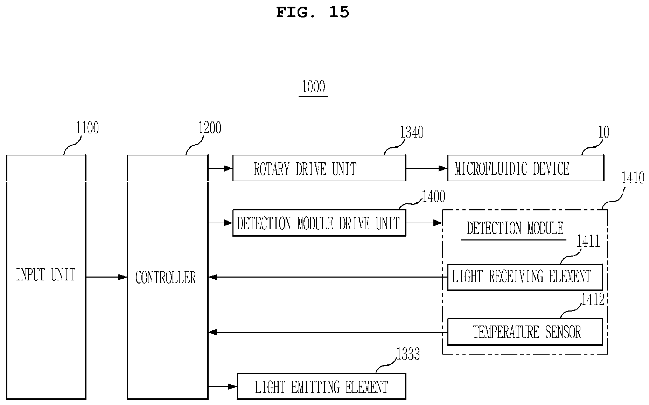

Hereinafter, a detailed description will be given of the test device according to an exemplary embodiment with reference to FIGS. 15 to 28.

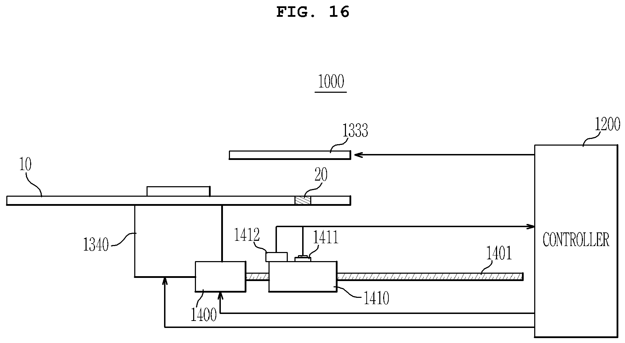

FIG. 15 is a block diagram showing the configuration of a test device according to an exemplary embodiment, and FIG. 16 is a conceptual side view illustrating the configuration of the test device according to the exemplary embodiment.

According to the illustrated exemplary embodiment, the test device 1000 includes a rotary drive unit 1340 to rotate the microfluidic device 10, a light emitting element 1333 to emit light to the microfluidic device 10, a detection module 1410 provided with a light receiving element 1411 to detect the detection object 20 through the light emitted from the light emitting element 1333 and a temperature sensor 1412 to sense the temperature of the detection object 20, a detection module drive unit 1400 to move the detection module 1410 in a radial direction relative to the microfluidic device 10, an input unit 1100 allowing a user to input a command therethrough from the outside, and a controller 1200 to control overall operations and functions of the test device 1000 according to the command inputted through the input unit 1100.

The microfluidic device 10 of the present exemplary embodiment is the same as the microfluidic device 10 described above with reference to FIGS. 1 to 4, and thus a detailed description thereof will be omitted.

When the microfluidic device 10 is loaded, the rotary drive unit 1340, which may include a spindle motor, is controlled by the controller 1200 to rotate the microfluidic device 10. The rotary drive unit 1340 may receive a signal outputted from the controller 1200 and repeat the operation of rotation and stop, thereby generating centrifugal force to transfer the fluid within the microfluidic device 10 or to move various structures on the microfluidic device 10 to desired positions.

Also, the rotary drive unit 1340 may include a motor drive to control the angular position of the microfluidic device 10. For example, the motor drive may employ a stepping motor or a DC motor.

The light emitting element 1333 may be a surface light source having a large light emitting area to uniformly emit light to a certain region of the microfluidic device 10. For example, a back light unit may be used as the light emitting element 1333.

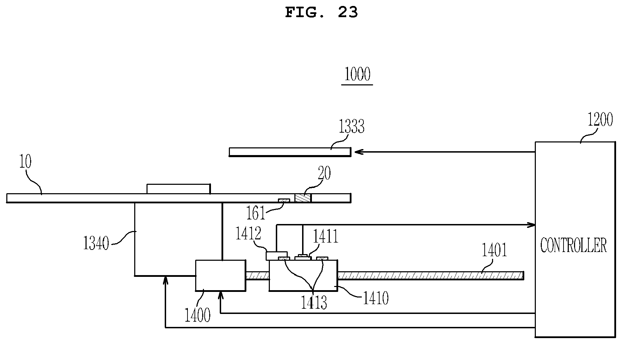

The light emitting element 1333 may be arranged to face the same direction as the light receiving element 1411, or it may be arranged to face the light receiving element 1411, as shown in FIG. 23. FIG. 23 shows that the light emitting element 1333 is positioned above the upper surface of the microfluidic device 10 and the light receiving element 1411 is positioned below the lower surface of the microfluidic device 10 such that the microfluidic device 10 is placed between the light emitting element 1333 and the light receiving element 1411. However, the positions of the light emitting element 1333 and the light receiving element 1411 may be changed. The light emitting element 1333 may be controlled by the controller 1200 to adjust the amount of light emitted therefrom.

The light receiving element 1411 receives light reflected from or transmitted through the detection object 20 after being emitted from the light emitting element 1333, thereby detecting the detection object 20. The light receiving element 1411 may be a complementary metal-oxide-semiconductor (CMOS) image sensor or a charge-coupled device (CCD) image sensor.

When the light receiving element 1411 receives light reflected from or transmitted through the detection object 20 and obtains an image of the detection object 20, the controller 1200 detects the presence of the detection object 20, e.g., the analyte in the test paper and the concentration of the analyte, using the image.

According to the illustrated exemplary embodiment, the test device 1000 has one light receiving element 1411 installed at the detection module 1410, which is a mechanism moveable in a radial direction, such that the light receiving element 1411 may detect a plurality of detection objects 20 provided in the microfluidic device 10.

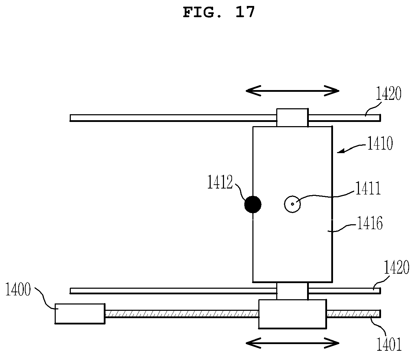

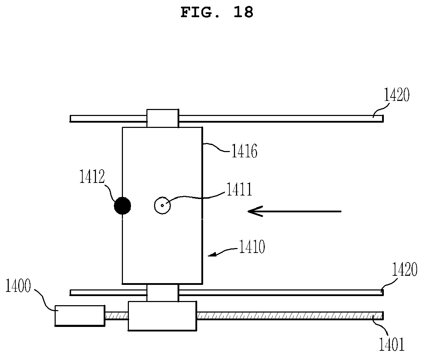

FIGS. 17 and 18 are views illustrating radial movement of a detection module 1410 according to an exemplary embodiment, which is seen from the top.

Referring to FIGS. 17 and 18, the detection module 1410 may be moved in a radial direction by driving force supplied from the detection module drive unit 1400. The detection module drive unit 1400 may be a feeding motor or stepping motor.

The travel distance of the detection module 1410 may be longer than the radius of the microfluidic device 10. The travel distance is sufficient if the detection module 1410 can move from beyond the outer circumference of the microfluidic device 10 to a position near the center of the microfluidic device 10.

The detection module 1410 may include a plate 1416 on which a constituent such as the light receiving element 1411 and/or temperature sensor 1412 is installed. The detection module 1410 may be slidably moved along two guide members 1420, which provide stable radial movement thereof. The guide members 1420 may be rod shaped, and the plate 1416 may be coupled to the guide members 1420 to allow for stable movement along the guide members 1420.

Also, the detection module 1410 is mounted on a power transmission part 1401 such that power produced by the detection module drive unit 1400 may be transmitted to the detection module 1410 to move the detection module 1410 in a radial direction. That is, when the detection module drive unit 1400 is actuated and the power thereof is transmitted to the detection module 1410 through the power transmission part 1401, the detection module 1410 moves along the power transmission part 1401 and the guide members 1420 in a radial direction.

As discussed above, FIG. 16 shows that the detection module 1410 is positioned under the microfluidic device 10 and the light emitting element 1333 is positioned over the microfluidic device 10. However, this configuration is simply an example and the positions of the detection module 1410 and the light emitting element 1333 may be changed.

A temperature sensor 1412 may be mounted on the detection module 1410 to detect the temperatures of the detection objects 20 arranged on different radii of the microfluidic device 10.

For the temperature sensor 1412, a non-contact temperature sensor such as an infrared temperature sensor, or a contact temperature sensor such as a thermistor, a resistance temperature detector and a thermocouple may be used. In case that the temperature of detection object 20 needs to be kept constant, a non-contact temperature sensor may be used to measure the temperature of the detection object 20 in a non-contact manner.

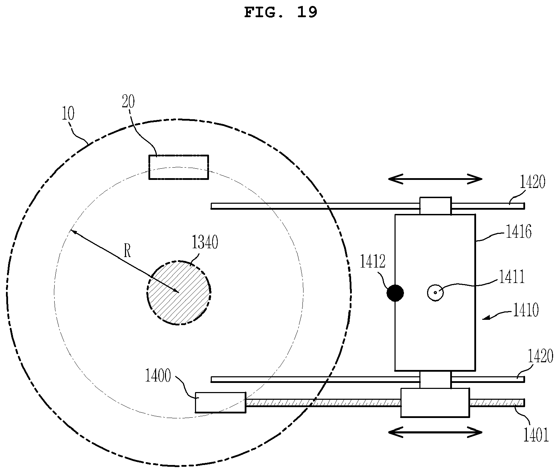

FIGS. 19 to 21 are views illustrating movement of the detection object 20 to a position facing the light receiving element 1411 of the detection module 1410 by movement of the detection module 1410 and rotation of the microfluidic device 10 in the test device 1000, according to an exemplary embodiment, which is viewed from the top.

The microfluidic device 10, which is positioned above the detection module 1410, is shown in a dotted line to more clearly show movement of the detection module 1410, which is positioned below the microfluidic device 10 and shown in a solid line.

FIG. 19 shows the microfluidic device 10 which has stopped rotating as the reaction in the microfluidic device 10 is completed. In FIG. 19, the detection module 1410 is positioned below the microfluidic device 10, beyond the outer circumference of the microfluidic device 10. For convenience of description, other structures in the microfluidic device 10 are not shown, and only one detection object 20 is shown.

Here, the detection object 20 may be test paper included in the reaction chamber 150 of the microfluidic device 10, as described above, to detect the presence of an analyte through chromatography.

When the reaction in the microfluidic device 10 is completed and rotation of the microfluidic device 10 is stopped, the controller 1200 controls the detection module drive unit 1400 to move the detection module 1410 in a radial direction such that the detection module 1410 is positioned at radius R at which the detection object 20 to be detected is positioned.

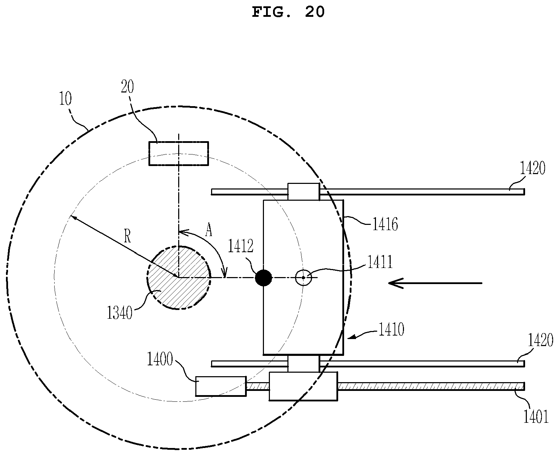

FIG. 20 illustrates that, after moving the detection module 1410 toward the center of the microfluidic device 10 in a radial direction, the light receiving element 1411 of the detection module 1410 has reached radius R at which the detection object 20 of the microfluidic device 10 is installed.

Information on radii at which the detection objects 20 provided in the microfluidic device 10 are installed may be pre-stored in the controller 1200. When actuating the detection module drive unit 1400, the controller 1200 controls the detection module drive unit 1400 using the information such that the light receiving element 1411 of the detection module 1410 is moved to radius R at which a detection object 20 to be detected is installed.

Once the light receiving element 1411 of the detection module 1410 is positioned at radius R, the controller 1200 controls the rotary drive unit 1340 to rotate the microfluidic device 10 such that the detection object 20 of the microfluidic device 10 is moved to a position facing the light receiving element 1411 of the detection module 1410.

The controller 1200 may then determine the angle A between the position of the detection object 20 of the microfluidic device 10 and that of the light receiving element 1411 of the detection module 1410, and control the rotary drive unit 1340 to rotate the microfluidic device 10 by angle A.

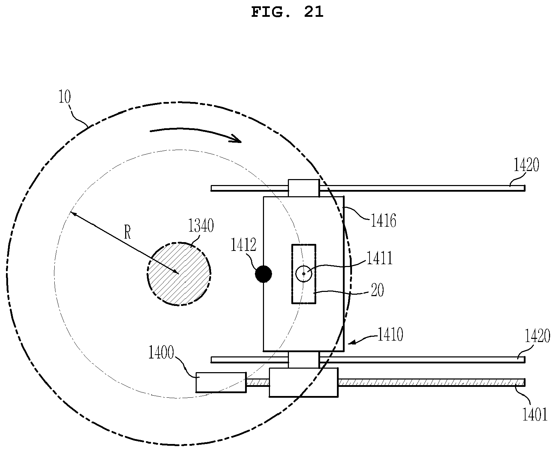

FIG. 21 shows the microfluidic device 10 after having been rotated such that the detection object 20 of the microfluidic device 10 is facing the light receiving element 1411 of the detection module 1410.

After the detection object 20 of the microfluidic device 10 has been moved to the position facing the light receiving element 1411 of the detection module 1410, the light emitting element 1333 is controlled by the controller 1200 to emit light to the detection object 20. The light receiving element 1411 then creates an image of the detection object 20 using the light emitted from the light emitting element 1333, which is transmitted through the detection object 20.

The controller 1200 computes the presence and/or concentration of the analyte by analyzing the created image of the detection object 20.

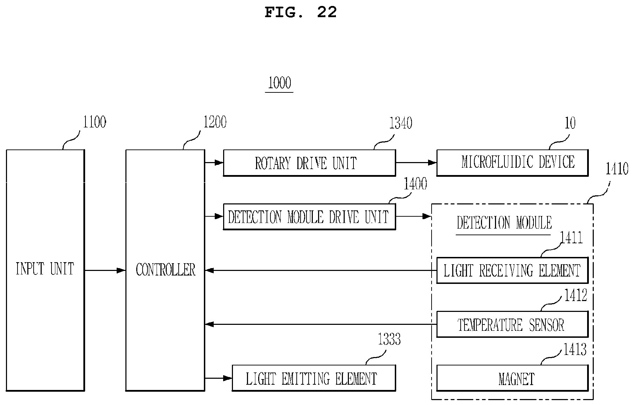

FIG. 22 is a block diagram illustrating a configuration of a test device 1000 according to another exemplary embodiment, and FIG. 23 is a conceptual side view illustrating the configuration of the test device 1000 according to the exemplary embodiment.

Referring to FIG. 22, the test device 1000 includes a rotary drive unit 1340 to rotate the microfluidic device 10, a light emitting element 1333 to emit light to the microfluidic device 10, a detection module 1410 provided with a light receiving element 1411 to detect the detection object 20 through the light emitted from the light emitting element 1333, at least one magnet 1413 to apply attractive force to the magnetic body 161 of the microfluidic device 10 and a temperature sensor 1412 to sense the temperature of the detection object 20, a detection module drive unit 1400 to move the detection module 1410 in a radial direction, an input unit 1100 allowing a user to input a command therethrough from the outside, and a controller 1200 to control overall operations and functions of the test device 1000 according to the command inputted through the input unit 1100.

In the illustrated exemplary embodiment, all of the details other than including the magnet 1413 in the detection module 1410 are the same as those in the previous exemplary embodiment illustrated in FIGS. 15 to 21, and thus a description thereof will be omitted, and the detection module 1410 having the magnets 1413 mounted thereto will be described in detail.

In the test device 1000 according to the illustrated exemplary embodiment, the detection module 1410 may include at least one magnet 1413, as shown in FIG. 23.

The magnet 1413 provided in the detection module 1410 applies attractive force to the magnetic body 161 within the magnetic body accommodating chamber 160 formed in a region adjacent to the detection object 20, in order to identify the position of the detection object 20 of the microfluidic device 10. In the illustrated exemplary embodiment, a magnet is accommodated in the detection module 1410, and a magnetic material is accommodated in the magnetic body accommodating chamber of the microfluidic device. However, embodiments of the present invention are not limited thereto, and the magnetic material may be accommodated in the detection module, with a magnet being accommodated in the magnetic body accommodating chamber of the microfluidic device 10.

When the magnet 1413 of the detection module 1410 and the magnetic body 161 of the microfluidic device 10 face each other, attractive force is applied to the magnetic body 161 by the magnet 1413, thereby keeping the microfluidic device 10 in place as long as force exceeding the attractive force is not applied thereto.

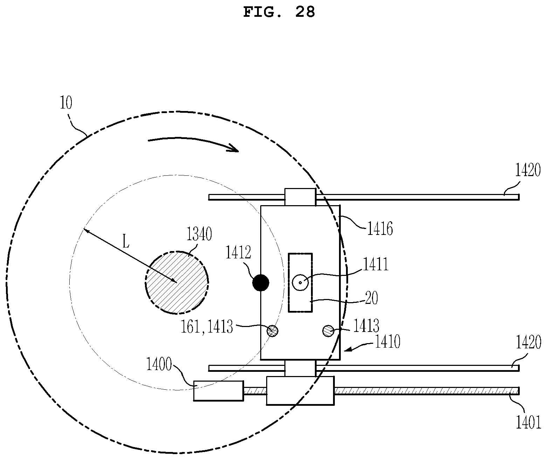

As shown in FIG. 28, when the magnetic body 161 of the microfluidic device 10 is positioned facing the magnet 1413 and the position thereof is fixed by the attractive force, the magnet 1413 in the detection module 1410 is arranged at a position at which the detection object 20 faces the light receiving element 1411 of the detection module 1410.

As such, with the magnet 1413 installed on the detection module 1410, when the magnetic body 161 is moved close to the magnet 1413 by rotation of the microfluidic device 10 toward the light receiving element 1411 to detect the detection object 20, the magnetic body 161 is fixed by the attractive force of the magnet 1413, and thereby the microfluidic device 10 stops moving, with the detection object 20 facing the light receiving element 1411.

Accordingly, the detection object 20 may be stably detected. For example, a shock sent to the detection object 20 from the outside during detection of the detection object 20 may interfere with exact detection of the detection object 20. However, the attractive force acting between the magnet 1413 and the magnetic body 161 to stably fix the microfluidic device 10 ensures stable detection of the detection object 20 .

In FIGS. 23 to 28, the detection module 1410 is provided with two magnets 1413. However, the number of the magnets is not limited thereto since the magnetic body 161 is moveable through rotation of the microfluidic device 10, while the detection module 1410 is moveable in a radial direction relative to the microfluidic device 10. Thus, the magnetic body 161 is moveable to the position facing the magnet 1413 of the detection module 1410 from any position on the microfluidic device 10.

However, in the illustrated exemplary embodiment, the detection module 1410 is provided with two magnets 1413 since two or more magnets 1413 allows other detection objects 20 at different radii on the microfluidic device 10 to make shorter movements to face the light receiving element 1411, as opposed to when only one magnet 1413 is provided.

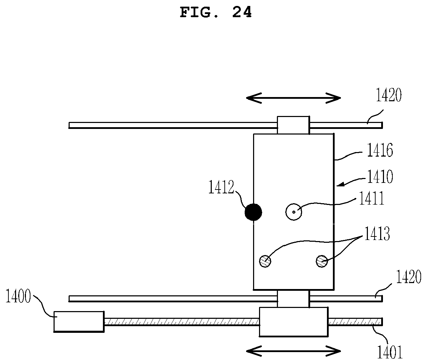

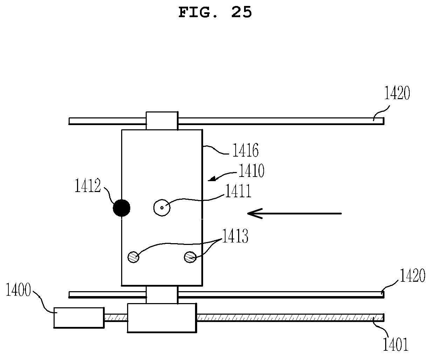

FIGS. 24 and 25 are views illustrating radial movement of the detection module 1410 according to an exemplary embodiment, in which the detection module 1410 is viewed from the top.

The configuration and operation of the detection module 1410 is the same as the detection module 1410 of FIGS. 17 and 18, except for the addition of magnets 1413 installed thereon, and thus a description thereof will be omitted.

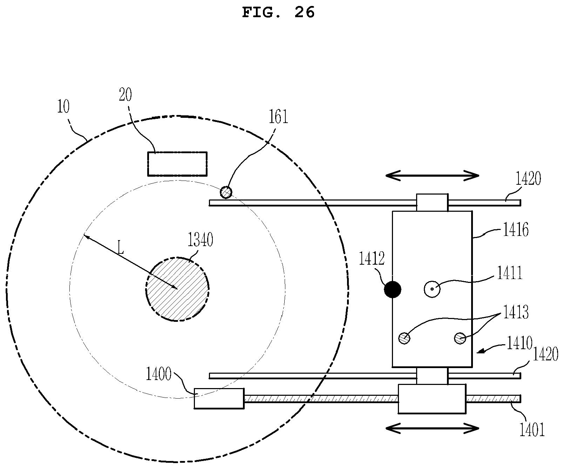

FIGS. 26 to 28 are views illustrating movement of a detection object 20 to a position facing the light receiving element 1411 of the detection module 1410 by movement of the detection module 1410 and rotation of the microfluidic device 10 in a test device 1000 according to another exemplary embodiment.

The microfluidic device 10, which is positioned above the detection module 1410, is shown in a dotted line to more clearly show movement of the detection module 1410, which is positioned below the microfluidic device 10 and shown in a solid line.

FIG. 26 shows the microfluidic device 10 which has stopped rotating as the reaction in the microfluidic device 10 is completed. In FIG. 26, the detection module 1410 is positioned below the microfluidic device 10, and beyond the outer circumference of the microfluidic device 10. For convenience of description and illustration, other structures in the microfluidic device 10 are not shown, and only one detection object 20 and one magnetic body 161 to identify the position of the detection object 20 is shown.

After the reaction in the microfluidic device 10 is completed and rotation of the microfluidic device 10 is stopped, the controller 1200 controls the detection module drive unit 1400 to move the detection module 1410 in a radial direction (denoted by arrows) such that one of the magnets 1413 of the detection module 1410 is positioned at radius L of the microfluidic device 10, at which the magnetic body 161 is disposed. The magnetic body 161 is located in a region adjacent to the detection object 20 to be detected.

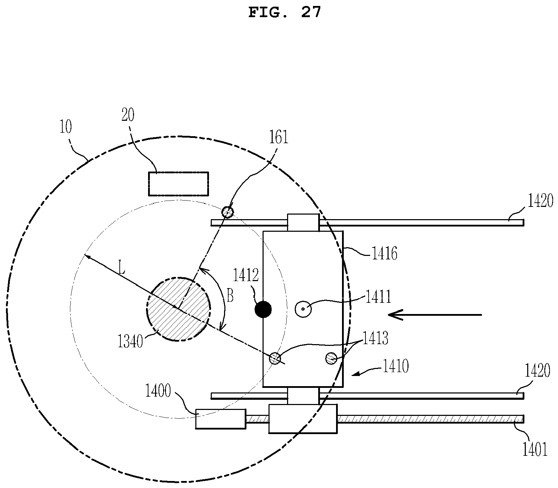

FIG. 27 illustrates that the magnet 1413 of the detection module 1410 has reached radius L of the microfluidic device 10 moving toward the center of the microfluidic device 10 in a radial direction.

Information on the radii at which the magnetic bodies 161 provided in the microfluidic device 10 are installed may be pre-stored in the controller 1200. Thus, the controller 1200 controls the detection module drive unit 1400 using the information in order to position the magnet 1413 of the detection module 1410 at radius L.

After the magnet 1413 of the detection module 1410 is positioned at radius L, the controller 1200 controls the rotary drive unit 1340 to rotate the microfluidic device 10 such that the magnetic body 161 of the microfluidic device 10 is moved to a position facing the magnet 1413 of the detection module 1410.

When the magnetic body 161 approaches the magnet 1413 of the detection module 1410, the magnetic body 161 is fixed at the position facing the magnet 1413 by the attractive force of the magnet 1413 applied to the magnetic body 161. Thereby, the microfluidic device 10 stops with the detection object 20 of the microfluidic device 10 facing the light receiving element 1411 of the detection module 1410.

FIG. 28 shows the magnetic body 161 of the microfluidic device 10 having been moved to the position facing the magnet 1413 of the detection module 1410 by rotation of the microfluidic device 10.

The controller 1200 may determine angle B between the magnetic body 161 of the microfluidic device 10 and the magnet 1413 of the detection module 1410 and control the rotary drive unit 1340 to rotate the microfluidic device 10 by angle B (Figure 27).

After the magnetic body 161 of the microfluidic device 10 moves to the position facing the magnet 1413 of the detection module 1410 and the microfluidic device 10 stops rotating, the light emitting element 1333 is controlled by the controller 1200 to emit light to the detection object 20. Then the light receiving element 1411 creates an image of the detection object 20 using the light emitted from the light emitting element 1333 and transmitted through the detection object 20.

The controller 1200 then determines the presence and/or concentration of the analyte by analyzing the created image of the detection object 20.

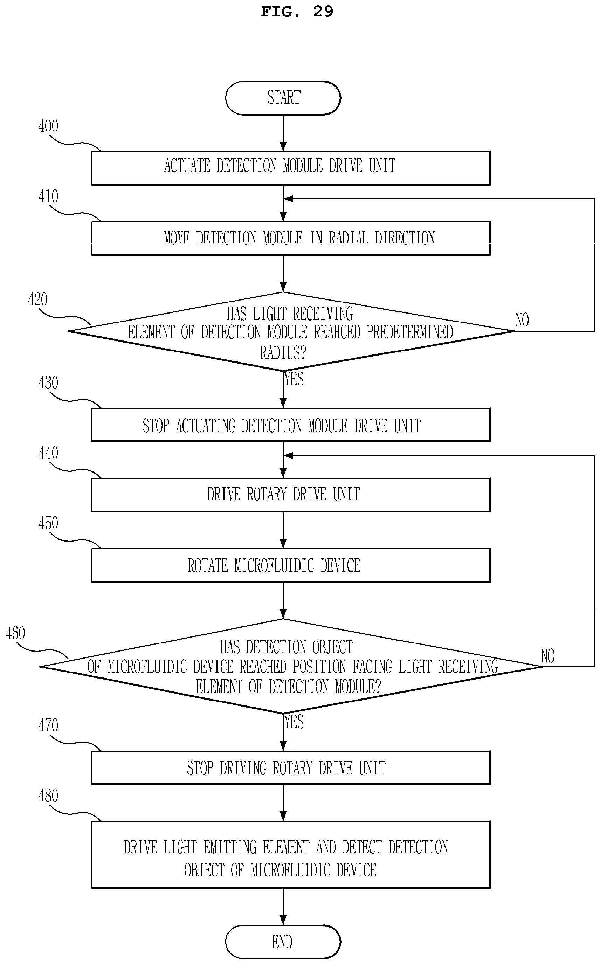

FIG. 29 is a flowchart illustrating a control method of the test device 1000 according to an exemplary embodiment.

Referring to FIG. 29, the controller 1200 first actuates the detection module drive unit 1400 (400), and moves the detection module 1410 in a radial direction (410).

After the reaction in the microfluidic device 10 is completed and rotation of the microfluidic device 10 is stopped, the controller 1200 controls the detection module drive unit 1400 to move the detection module 1410 in a radial direction such that the light receiving element 1411 of the detection module 1410 is positioned at the radius at which the detection object 20 to be detected is installed.

The controller 1200 determines whether the light receiving element 1411 of the detection module 1410 has reached the predetermined radius (420), and if the light receiving element 1411 of the detection module 1410 has reached the predetermined radius, the controller 1200 stops actuation of the detection module drive unit 1400 (430).

Here, the predetermined radius represents radius R on the microfluidic device 10 at which the detection object 20 provided in the microfluidic device 10 is installed. Information on the radii at which the detection objects 20 provided in the microfluidic device 10 are installed may be pre-stored in the controller 1200. When actuating the detection module drive unit 1400, the controller 1200 controls the detection module drive unit 1400 using the information such that the light receiving element 1411 of the detection module 1410 is moved to radius R to enable detection of the detection object 20.

When the light receiving element 1411 of the detection module 1410 is positioned at radius R and the detection module drive unit 1400 stops, the controller 1200 actuates the rotary drive unit 1340 (440), thereby rotating the microfluidic device 10 (450).

That is, the controller 1200 controls the rotary drive unit 1340 to rotate the microfluidic device 10 such that the detection object 20 of the microfluidic device 10 is moved to a position facing the light receiving element 1411 of the detection module 1410.

The controller 1200 determines angle A between position of the detection object 20 of the microfluidic device 10 and that of the light receiving element 1411 of the detection module 1410, and controls the rotary drive unit 1340 to rotate the microfluidic device 10 by angle A.

The controller 1200 determines whether the detection object 20 of the microfluidic device 10 has reached the position facing the light receiving element 1411 of the detection module 1410 (460), and if the detection object 20 has reached the position facing the light receiving element 1411 of the detection module 1410, the controller 1200 stops actuation of the rotary drive unit 1340 (470).

After the detection object 20 reaches the position facing the light receiving element 1411 of the detection module 1410 and the controller 1200 stops actuating the rotary drive unit 1340, the microfluidic device 10 stops moving, with the detection object 20 of the microfluidic device 10 facing the light receiving element 1411 of the detection module 1410.

When the detection object 20 of the microfluidic device 10 reaches the position facing the light receiving element 1411 of the detection module 1410 and the microfluidic device 10 stops rotating as actuation of the rotary drive unit 1340 is stopped, the controller 1200 actuates the light emitting element 1333 to detect the detection object 20 of the microfluidic device 10 (480).

The controller 1200 drives the light emitting element 1333 to emit light onto the detection object 20, and the light receiving element 1411 creates an image of the detection object 20 using the light emitted from the light emitting element 1333 and transmitted through the detection object 20. The controller 1200 then determines the presence and/or concentration of the analyte by analyzing the created image of the detection object 20.

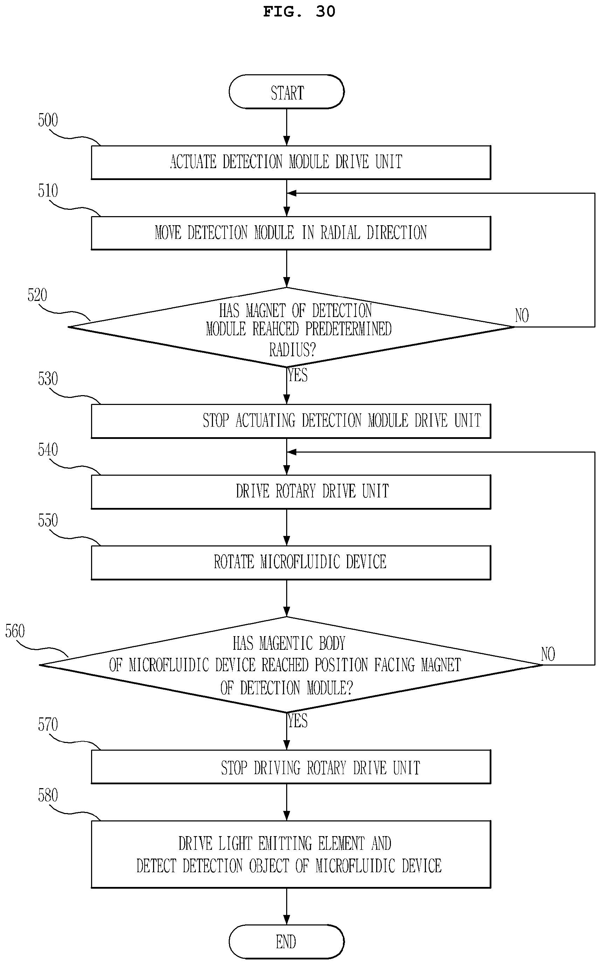

FIG. 30 is a flowchart illustrating a control method of the test device 1000 according to an exemplary embodiment.

Referring to FIG. 30, the controller 1200 first actuates the detection module drive unit 1400 (500), and moves the detection module 1410 in a radial direction (510).

After the reaction in the microfluidic device 10 is completed and rotation of the microfluidic device 10 is stopped, the controller 1200 controls the detection module drive unit 1400 to move the detection module 1410 in a radial direction such that one of the magnets 1413 of the detection module 1410 is positioned at radius L of the microfluidic device 10. Radius L denotes the position at which the magnetic body 161 installed in a region adjacent to the detection object 20 to be detected is positioned on the microfluidic device 10.

The controller 1200 determines whether the magnet 1413 of the detection module 1410 has reached the predetermined radius (520), and if the magnet 1413 of the detection module 1410 has reached the predetermined radius, the controller 1200 stops actuation of the detection module drive unit 1400 (530).

As described above, the predetermined radius represents radius L on the microfluidic device 10 at which the magnetic body 161 provided in the microfluidic device 10 is installed. Information on the radii at which the magnetic bodies 161 provided in the microfluidic device 10 are installed may be pre-stored in the controller 1200. The controller 1200 controls the detection module drive unit 1400 using the information such that the magnet 1413 of the detection module 1410 is moved to a position corresponding to radius L to enable detection of detection object 20.

After the magnet 1413 of the detection module 1410 is positioned at radius L and the detection module drive unit 1400 stops, the controller 1200 actuates the rotary drive unit 1340 (540) to rotate the microfluidic device 10 (550).

That is, the controller 1200 controls the rotary drive unit 1340 to rotate the microfluidic device 10 such that the magnetic body 161 of the microfluidic device 10 is moved to the position facing the magnet 1413 of the detection module 1410. The controller 1200 may determine angle B between the position of the magnetic body 161 of the microfluidic device 10 and that of the magnet 1413 of the detection module 1410, and control the rotary drive unit 1340 to rotate the microfluidic device 10 by angle B.

The controller 1200 determines whether magnetic body 161 of the microfluidic device 10 has reached the position facing the magnet 1413 of the detection module 1410 (560), and if the magnetic body 161 has reached the position facing the magnet 1413 of the detection module 1410, the controller 1200 stops actuation of the rotary drive unit 1340 (570).

After the magnetic body 161 reaches the position facing the magnet 1413 of the detection module 1410 and the controller 1200 stops actuating the rotary drive unit 1340, the magnetic body 161 is fixed at the position facing the magnet 1413 by attractive force of the magnet 1413 applied to the magnetic body 161. Thus, the microfluidic device 10 stops with the detection object 20 of the microfluidic device 10 facing the light receiving element 1411 of the detection module 1410.

When the magnetic body 161 of the microfluidic device 10 reaches the position facing the magnet 1413 of the detection module 1410 and actuation of the rotary drive unit 1340 is stopped to stop rotation of the microfluidic device 10, the controller 1200 drives the light emitting element 1333 to detect the detection object 20 of the microfluidic device 10 (580).

The light emitting element 1333 is controlled by the controller 1200 to emit light onto the detection object 20, and the light receiving element 1411 creates an image of the detection object 20 using the light emitted from the light emitting element 1333 and transmitted through the detection object 20. The controller 1200 then determines the presence and/or concentration of the analyte by analyzing the created image of the detection object 20.

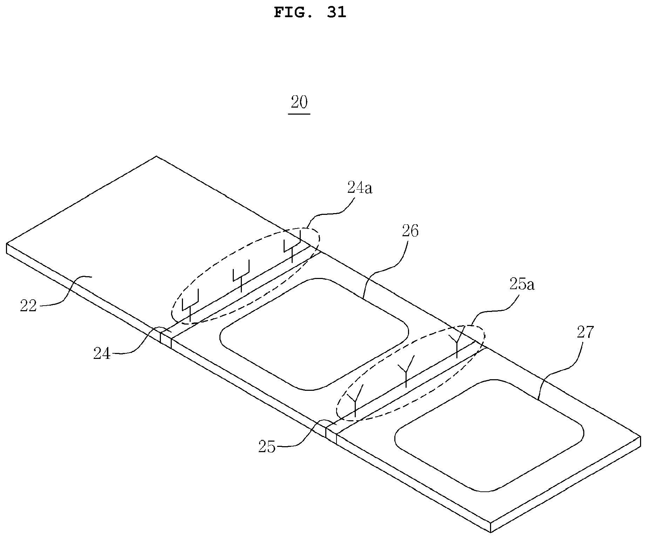

FIG. 31 is a view schematically illustrating a test paper used as an example of the detection object 20 of a test device according to an exemplary embodiment.

The detection object 20 includes a test paper-type object formed of micropore, micro pillar, or thin porous membrane such as cellulose, thereby allowing capillary pressure to act. Referring to FIG. 31, a sample pad 22 on which the sample is applied is formed at one end of the detection object 20, and a test line 24, on which a first capture material 24a to detect an analyte, is immobilized.

When a biosample such as blood or urine is dropped on the sample pad 22, the biosample flows to the opposite side due to the capillary pressure. For example, if the analyte is antigen Q and binding between the analyte and the first marker conjugate occurs in the second chamber 130, the biosample will contain a conjugate of antigen Q and the first marker conjugate.

In case that the analyte is antigen Q, the capture material 24a immobilized on the test line 24 may be antibody Q. When the biosample flowing according to the capillary pressure reaches the test line 24, the conjugate of antigen Q and the first marker conjugate binds with antibody Q 24a, thereby forming a sandwich conjugate. Therefore, if the analyte is contained in the biosample, it may be detected by the marker on the test line 24.

If the amount of the sample is small or the sample is contaminated, a standard test often fails due to various causes. Accordingly, to determine whether the test has been properly performed, the detection object 20 is provided with a control line 25 on which is immobilized a second capture material 25a that specifically reacts with a material contained in the sample regardless of presence of the analyte. As described above with reference to FIG. 2, the sample that has passed the second chamber 130 contains the second marker conjugate having a certain material which specifically reacts with the second capture material 25a. Therefore, if the sample has properly flowed on the detection object 20, the second marker conjugate will bind with the second capture material 25a on the control line 25 and the binding will be marked by the marker.

For the second capture material 25a immobilized on the control line 25, biotin may be used, and thus the second marker conjugate carried by the sample in the second chamber 130 may be a streptavidin-marker conjugate, which has a high affinity for biotin.

If the sample is normally moved to the opposite side by osmotic pressure, the second marker conjugate also moves with the sample. Accordingly, regardless of the presence of the analyte in the sample, a conjugate is formed by conjugation between the second marker conjugate and the second capture material 25a, and marked on the control line 25 by the marker.

In other words, if a mark by the marker appears on both the control line 25 and the test line 24, the sample will be determined as positive, which indicates that the analyte is present in the sample. If the mark appears only on the control line 25, the sample will be determined as negative, which indicates that the analyte is not present in the sample. On the contrary, if the mark does not appear on the control line 25, it may be determined that the test has not been properly performed.

The light receiving element 1411 captures an image of the test paper described above through the light emitted from the light emitting element 1333. A signal value of the test line 24 may be obtained from the captured image to detect the presence and/or concentration of the analyte.

Depending on the condition of the test paper, the brightness of the test paper may vary from test to test. This may lead to variation in assay results, and thus there is a need to maintain uniform brightness of the test paper.

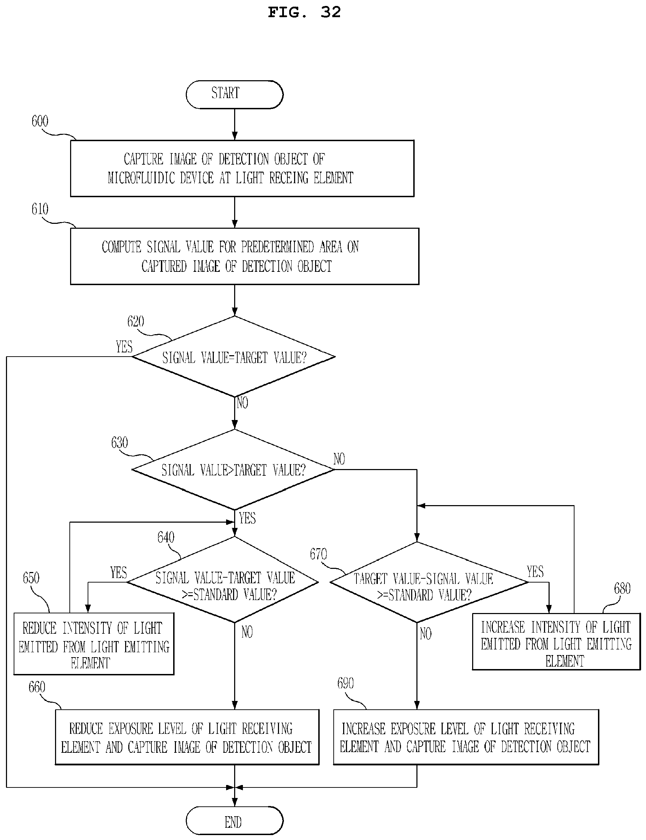

FIG. 32 is a flowchart illustrating a control method of a test device 1000 according to an exemplary embodiment to capture an image of the detection object 20, i.e., test paper, while maintaining uniform brightness of the test paper in the test device 1000.

Referring to FIG. 32, the light receiving element 1411 first captures an image of the detection object 20, i.e., test paper of the microfluidic device 10 (600). The light receiving element 1411 transmits the captured image to the controller 1200.

The controller 1200 computes a signal value for a predetermined area of the detection object 20 (610).

That is, the controller 1200 computes a signal value for the predetermined area from the image of the test paper transmitted from the light receiving element 1411.

Here, the predetermined area may include, as shown in FIG. 31, an area between the test line 24 and the control line 25 (a first area 26) or an area beyond the control line 25 (a second area 27).

However, these exemplary embodiments are not limited thereto, and a signal value may be computed for a different area on the test paper. It should be understood that the signal values of the test line 24 and the control line 25 are important for analysis of the reaction result, and thus a signal value may be computed for the area between the test line 24 and the control line 25 (first area 26) or the area beyond the control line 25 (second area 27).

Here, for the signal value, an RGB signal value, a YCbCr signal value or a Gray-scale signal value may be used.

After the signal value is computed, the controller 1200 determines whether the computed signal value is equal to a predetermined target value (620). Here, one of the signal value of the first region 26 and the signal value of the second region 27 may be compared with a target value, or an average of the signal values of the first region 26 and the second region 27 may be compared with the target value. Hereinafter for explanation purposes, a description will be given of a case in which the signal value of the first region 26 is compared with the target value.

Here, the target value, which is a value that the computed signal value should comply with, reflects the optimum brightness targeted in capturing an image of the test paper. The target value may be determined through a repeated experiment and pre-stored in the controller 1200.

The controller 1200 compares the computed signal value with the target value, and depending on the result of comparison, it controls the light receiving element 1411 and/or the light emitting element 1333 to adjust the brightness of the test paper to ensure that an image of the test paper is captured with a uniform brightness.

If the signal value is equal to the target value, the control operation is terminated since a separate adjustment of brightness is unnecessary.

If the signal value is different from the target value, the controller 1200 determines if the signal value exceeds the target value (630). If the signal value exceeds the target value, the controller 1200 determines that the brightness of the test paper is higher than the optimum brightness and performs a control operation to capture a darker image of the test paper.

That is, if the signal value is greater than the target value, the controller 1200 determines whether the difference between the signal value and the target value is greater than or equal to a predetermined standard value (640).

If the difference between the signal value and the target value is greater than or equal to the predetermined standard value, the controller 1200 controls the light emitting element 1333 to lower the intensity of light emitted from the light emitting element 1333 (650).

When the difference between the signal value and the target value is lowered below the predetermined standard value through this operation, the controller 1200 may reduce the exposure level of the light receiving element 1411 to capture a darker image of the detection object 20 (660).

If the signal value is greater than the target value, it means that the brightness of the test paper is higher than the optimum brightness reflected on the target value, and thus to obtain a detection result having a lowered brightness variation, a darker image of the test paper may need to be captured.

If the signal value is greater than the target value and the different therebetween is greater than or equal to the predetermined standard value, the controller 1200 determines that the difference between the signal value and the target value is large.

That is, the controller 1200 determines that a control operation needs to be performed to reduce the intensity of light emitted from the light emitting element 1333 to a certain level prior to a fine adjustment of the brightness through control of the exposure level of the light receiving element 1411.

Here, the standard value is a value reflecting the difference between the signal value and the target value as large, thereby requiring that the light intensity of the light emitting element 1333 be reduced prior to adjustment of the brightness of the image though control of the exposure level of the light receiving element 1411. The standard value may be determined in a repeated experiment and pre-stored in the controller 1200.

As described above, the controller 1200 can reduce the intensity of light emitted from the light emitting element 1333 to a certain level if the signal value is greater than the target value, and the difference therebetween is greater than or equal to the standard value. Once the difference between the signal value and the target value is lowered below the standard value, the controller 1200 reduces the exposure level of the light receiving element 1411, and captures an image of the detection object 20.

The controller 1200 may control at least one of shutter speed and iris opening of the light receiving element 1411 to reduce the exposure level of the light receiving element 1411, i.e., to reduce the intensity of light received from the light receiving element 1411, to capture an image of the detection object 20.

The relationship between the variation in the exposure level of the light receiving element 1411 and the change in the signal value may be pre-established and pre-stored in the controller 1200. Thus, with reference to this relationship, the controller 1200 is able to compute an exposure level of the light receiving element 1411 in which the signal value is set to be equal to the target value, and accordingly, reduce the exposure level of the light receiving element 1411.

In operation 630, if the signal value is less than the target value, the controller 1200 determines that the brightness of the test paper is lower than the optimum brightness, and performs a control operation to capture a brighter image of the test paper.

That is, if the signal value is less than the target value, the controller 1200 determines whether the difference between the target value and the signal value is greater than or equal to the predetermined standard value (670).

If the difference between the target value and the signal value is greater than or equal to the predetermined standard value, the controller 1200 controls the light emitting element 1333 to increase the intensity of light emitted from the light emitting element 1333 (680).

Once the difference between the target value and the signal value is lowered below the predetermined standard value, the controller 1200 increases the exposure level of the light receiving element 1411 and captures a brighter image of the detection object 20 (690).

If the signal value is less than the target value, it means that the brightness of the test paper is lower than the optimum brightness reflected in the target value, and thus, a brighter image of the test paper may need to be captured to obtain a detection result having a lowered brightness variation.

If the signal value is less than the target value, and the difference between the target value and the signal value is greater than or equal to the predetermined standard value, the controller 1200 determines that the difference between the signal value and the target value is large.

That is, the controller 1200 determines that a control operation needs to be performed to increase the intensity of light emitted from the light emitting element 1333 to a certain level prior to a fine adjustment of the brightness through control of the exposure level of the light receiving element 1411.

Here, the standard value is a value reflecting the difference between the signal value and the target value as large, thereby requiring the light intensity of the light emitting element 1333 to be increased prior to adjustment of the brightness of an image of the test paper though control of the exposure level of the light receiving element 1411. The standard value may be determined in a repeated experiment and pre-stored in the controller 1200. The standard value of operation 670 may be set to be equal to that of operation 640.

As described above, the controller 1200 increases the intensity of light emitted from the light emitting element 1333 to a certain level if the signal value is less than the target value, and the difference therebetween is greater than or equal to the standard value,. Once the difference between the target value and the signal value is lowered below the standard value, the controller 1200 increases the exposure level of the light receiving element 1411 and captures an image of the detection object 20.

As discussed above, the controller 1200 may control at least one of shutter speed and iris opening of the light receiving element 1411 to increase the exposure level of the light receiving element 1411, i.e., to increase the intensity of light received from the light receiving element 1411 to capture an image of the detection object 20.

The relationship between the variation in the exposure level of the light receiving element 1411 and the change in the signal value may be pre-established and pre-stored in the controller 1200, and the controller 1200 computes. Thus, with reference to this relationship, the controller 1200 is able to compute an exposure level of the light receiving element 1411 in which the signal value is set to be equal to the target value, and accordingly, increases the exposure level of the light receiving element 1411.

As is apparent from the above description, variation in assay results of a detection object may be reduced.

Also, reliability of the assay results may be enhanced by reduction of variation in the assay results.

Although a few exemplary embodiments have been shown and described, it would be appreciated by those skilled in the art that changes may be made in these embodiments without departing from the principles and spirit of the present disclosure, the scope of which is defined in the claims and their equivalents.

## Claims

1. Test device (1000) for detecting a detection object (20) of a microfluidic device (10), comprising:
- a housing (1010) forming an external appearance of the test device (1000);
- an input unit (1100) configured to receive a command input by a user;
- a controller (1200) configured to control operation of the test device (1000) according to a command from the input unit (1100);
- a tray (1320) positioned in the housing (1010) and movable to a first state in which the tray (1320) is positioned outside the test device (1000) and to a second state in which the tray (1320) is positioned inside the test device (1000), wherein the tray (1320) is configured to receive a microfluidic device (10);
- a vertical movement part (1310) positioned in the housing (1010) and configured to perform a first movement during which the vertical movement part (1310) moves to an upper side at which the tray (1320) is arranged and a second movement which is performed in the direction opposite to the first movement, such that a microfluidic device (10) may be correctly seated on the tray (1320);
- an opening-closing drive unit (1300) configured to drive the vertical movement part (1310) and/or the tray (1320) according to a command from the controller (1200);
- a detection module (1410) including a light receiving element (1411);
- a detection module drive unit (1300) configured to drive the detection module (1410) according to a command from the controller (1200);
- a light emitting element (1333); and
- a rotary drive unit (1340) comprising a spindle motor configured to rotate a microfluidic device (10),
wherein the controller (1200) is configured to control
- operations related to the rotary drive unit (1340) of the test device 1000, providing a signal output to the rotary drive unit (1340) to repeat the operation of rotating and stopping a microfluidic device (10) to generate centrifugal force to transfer fluid within the microfluidic device (10) or to move various structures in the microfluidic device (10) to desired positions;
- operations of the tray (1320) and the vertical movement part (1310) to mount a microfluidic device (10) on the test device (1000); and
- operation of the detection module (1410) to perform a function related to detection of a detection object (20) in the microfluidic device (10).

2. The test device (1000) according to claim 1, wherein the rotary drive unit (1340) includes a motor drive to control the angular position of a microfluidic device (10).

3. The test device (1000) according to claim 1 or 2, wherein the detection module (1410) includes at least one magnet (1413) to guide a microfluidic device (10) including microfluidic structures and a magnetic body (161), a detection object (20) being positioned in at least one of the microfluidic structures, to the exact position of the microfluidic device (10).

4. The test device (1000) according to any of the preceding claims, wherein the detection module (1410) is movable within the housing (1010) of the test device (1000), the movement of the detection module (1410) performed in a direction in which the tray (1320) is accommodated in the housing (1010) being defined as a third movement, and the movement thereof performed in the direction opposite to the third movement being defined as a fourth movement, the third movement and the fourth movement being performed in a radial direction relative to a microfluidic device (10) when the microfluidic device (10) is seated on the tray (1320).

5. The test device (1000) according to any of the preceding claims, wherein the vertical movement part (1310) is arranged at the lower side of the tray (1320)

6. The test device (1000) according to any of the preceding claims, wherein the housing (1010) includes an upper housing and a lower housing, the light emitting element (1333) being positioned at the upper housing and the vertical movement part (1310) being positioned at the lower housing.

7. The test device (1000) according to claim 6, wherein the tray (1320) is be accommodated in the lower housing when in the second state

8. The test device (1000) according to any of the preceding claims, comprising a clamper (1330) positioned at the housing (1010), the clamper (1330) configured to press a microfluidic device (10) by force acting between the clamper (1330) and the rotary drive unit (1340).

9. The test device (1000) according to claim 8 and claim 6 or 7, wherein the upper housing (1010) is positioned at the upper side of the tray (1320) and the upper housing (1010) is provided with the clamper (1330) configured to fix a microfluidic device (10) to the rotary drive unit (1340).

10. The test device (1000) according to claim 9, wherein the clamper (1330) includes an accommodation portion (1331) to accommodate the rotary drive unit (1340) and a protrusion (1332) protruding to form an outer circumference that protrudes toward the surface of the microfluidic device (10) when a microfluidic device (10) is loaded onto the tray (1320), to be firmly coupled to the rotary drive unit (1340).

11. The test device (1000) according to claim 10, wherein
- the accommodation portion (1331) of the clamper (1330) is provided with a magnetic body, and a magnet is provided at the upper portion of the rotary drive unit (1340), or
- the accommodation portion (1331) of the clamper (1330) is provided with a magnet, and a magnetic body is provided at the upper portion of the rotary drive unit (1340).

12. The test device (1000) according to any of the preceding claims, wherein the detection module (1410) is arranged at the vertical movement part (1310).

13. The test device (1000) according to any of the preceding claims, wherein the tray (1320) is provided with a seating surface (1321) configured to have a microfluidic device (10) seated thereon.

14. The test device (1000) according to any of the preceding claims, wherein the vertical movement part (1310) is provided with the rotary drive unit (1340) which is configured to be inserted into a through hole of a microfluidic device (10) through an opening of the tray (1320), the rotary drive unit (1340) being configured to be coupled to the microfluidic device (10) to rotate the microfluidic device (10).

15. The test device (1000) according claim 13, comprising a head portion (1311) provided at the upper side of the rotary drive unit (1340) and configured to couple to the microfluidic device (10).
